# EUROPEAN PATENT APPLICATION

(11) **EP 1 118 622 A1**
(43) Date of publication of application: **25.07.2001**
(21) Application number: 99969723.8
(22) Date of filing: 29.09.1999
(51) Int. Cl.: C07K 16/40, C12P 21/08, G01N 33/53, A61K 39/395

(54) **NOVEL ANTIBODIES, DRUGS CONTAINING THESE ANTIBODIES AND METHODS FOR SCREENING COMPOUNDS BY USING THESE ANTIBODIES**

(30) Priority: 29.09.1998 JP 29150198; 29.09.1998 JP 29150398
(71) Applicant: KYOWA HAKKO KOGYO CO., LTD., Chiyoda-ku, Tokyo 100-8185 (JP)
(72) Inventor: HANAI, Nobuo, Sagamihara-shi, Kanagawa 229-0011 (JP); FURUYA, Akiko, Machida-shi, Tokyo 194-0033 (JP)
(74) Representative: VOSSIUS & PARTNER
(86) International application number: JP9905350
(87) International publication number: WO0018805

(57) **Abstract**

The present invention provides antibodies which recognize a novel, membrane-type matrix metalloproteinase polypeptide [MT4-MMP(2)] that has, different from the hitherto reported MT4-MMP, physiological activity; prophylactic agents, diagnostic agents and therapeutic agents comprising the antibodies, for various diseases such as arthrosis deformans, rheumatoid arthritis, asthma, autoimmune diseases, or atopic dermatitis; and methods of screening for inhibitors and activators of MT4-MMP(2) using the antibodies.

The second object of the present invention is to provide antibodies which recognize novel, human and mouse membrane-type matrix metalloproteinase MT5-MMP polypeptides having physiological activity; prophylactic agents, diagnostic agents and therapeutic agents comprising the antibodies, for various diseases such as arthrosis deformans, rheumatoid arthritis, asthma, autoimmune diseases, atopic dermatitis, brain disorders at the time of cerebral apoplexy, or Alzheimer's disease; and methods of screening for inhibitors and activators of MT5-MMP using the antibodies.

## Description

### TECHNICAL FIELD

The present invention relates to antibodies that specifically react with novel membrane-type matrix metalloproteinase polypeptides; pharmaceuticals containing the antibodies; and methods of screening for compounds that modulate the expression of the polypeptides or bind to the polypeptides using the antibodies.

### BACKGROUND ART

A group of enzymes generically termed "matrix metalloproteinases" (hereinafter, abbreviated to MMPs) that have metal ions at the active center are involved in the degradation of extracellular matrix composed of complicated components such as collagens, fibronectin, laminin and proteoglycans.

To date, the following MMPs have been reported: interstitial collagenase (MMP-1), gelatinase A (MMP-2), gelatinase B (MMP-9), stromelysin 1 (MMP-3), matrilysin (MMP-7), neutrophil collagenase (MMP-8), stromelysin 2 (MMP-10), stromelysin 3 (MMP-11), metallo-elastase (MMP-12), collagenase 3 (MMP-13), membrane type 1 MMP (MT1-MMP or MMP-14), membrane type 2 MMP (MT2-MMP or MMP-15), membrane type 3 MMP (MT3-MMP or MMP-16), membrane type 4 MMP (MT4-MMP or MMP-17), etc. (Protein, Nucleic Acid and Enzyme, 42, 2386 (1997)). These MMPs form a family, and each MMP is basically composed of an N-terminal propeptide domain, an active domain to which zinc ions bind, and a hemopexin-like domain. In MMP-7, no hemopexin-like domain is found. Membrane-type MMPs have a transmembrane domain and a intracellular domain at the C-terminal of the hemopexin-like domain.

A human MT4-MMP gene has already been reported [Puente, Cancer Research, 56, 944 (1996)]. However, a translation initiation site is not included in the nucleotide sequence of this gene, and this gene was defined as a human MT4-MMP gene simply because it comprises a nucleotide sequence containing MMP-like domains. Thus, it is difficult to consider that this gene encodes the full-length of MT4-MMP.

On the other hand, it is known that production of MT1-MMP is enhanced in patients with arthrosis deformans [Am. J. Pathol., 151, 245 (1997)]; that MMPs are important for the infiltration of leukocytes into tissues that is important in immunological and inflammatory reactions [J. Immunol., 156, 1 (1996)]; that MMP inhibitors prevent hepatitis [Eur. J. Pharmacol., 341, 105 (1998)]; and that MMP inhibitors are effective for treating corneal ulcer [Japanese Journal of Ophthalmology, 102, 270 (1998)].

It is also known that MMPs are important for cancer proliferation, infiltration and metastasis [Protein, Nucleic Acid and Enzyme, 42, 2386 (1997)], and it is reported that MMP inhibitors have carcinostatic activity [SCRIP, 2349, 20 (1998)].

Furthermore, it is suggested that MT4-MMP is expressed in leukocytes and thus may be involved in the migration and infiltration of leukocytes.

From what have been described so far, MMPs may be used for marker for diagnosing arthrosis deformans, rheumatoid arthritis, asthma, autoimmune diseases, atopic dermatitis, psoriasis, contact dermatitis, alopecia, ischemic heart diseases, immune response associated with organ transplantation, hepatitis, nephritis, pancreatitis, arteriosclerosis, leukemia, malignant tumor, wounds, corneal ulcer, tissue injury or inflammations associated with infiltration of leukocytes, brain disorders at the time of cerebral apoplexy, Alzheimer's disease, dementia, multiple sclerosis, Parkinson's disease or degree of malignancy of brain tumor, and inhibitors of MMPs are useful for preventing or treating these diseases.

The already reported MT4-MMP gene [Cancer Research, 56, 944 (1996)] does not include a transcription initiation site nor has such a domain structure as seen in known membrane-type MMPs such as MT1-MMP. Therefore, this gene represents a sequence encoding a non-physiological peptide not expressed *in vivo.*

The first object of the present invention is to provide antibodies which recognize a novel membrane-type matrix metalloproteinase polypeptide [hereinafter, sometimes abbreviated to MT4-MMP(2)] that is, different from the hitherto reported MT4-MMP, physiologically active; prophylactic agents, diagnostic agents and therapeutic agents containing the antibodies for arthrosis deformans, rheumatoid arthritis, asthma, autoimmune diseases, atopic dermatitis, psoriasis, contact dermatitis, alopecia, ischemic heart diseases, brain disorders at the time of cerebral apoplexy, organ transplantation, hepatitis, nephritis, pancreatitis, arteriosclerosis, leukemia, malignant tumor, wounds, corneal ulcer, tissue injury, or inflammations associated with infiltration of leukocytes; and methods of screening for inhibitors or activators of MT4-MMP(2) using the antibodies.

The second object of the present invention is to provide antibodies which recognize novel, human and mouse membrane-type matrix metalloproteinase MT5-MMP polypeptides that are physiologically active; prophylactic agents, diagnostic agents and therapeutic agents containing the antibodies for arthrosis deformans, rheumatoid arthritis, asthma, autoimmune diseases, atopic dermatitis, psoriasis, contact dermatitis, alopecia, ischemic heart diseases, immune response associated with organ transplantation, hepatitis, nephritis, pancreatitis, arteriosclerosis, leukemia, malignant tumor, wounds, corneal ulcer, tissue injury, inflammations associated with infiltration of leukocytes, brain disorders at the time of cerebral apoplexy, Alzheimer's disease, dementia, multiple sclerosis, Parkinson's disease or brain tumor; and methods of screening for inhibitors or activators of MT5-MMP using the antibodies.

### DISCLOSURE OF THE INVENTION

The present inventors have made intensive and extensive researches based on the assumption that the known human MT4-MMP is not a protein having the inherent activity of MT4-MMP and that a true MT4-MMP protein having the activity should exist. As a result, the inventors have found a novel and true MT4-MMP (hireinafter, referred to as "MP4-MMP(2)) polypeptide and succeeded in obtaining antibodies that recognize the polypeptide. Thus, the present invention has been achieved.

Also, the present inventors have made intensive and extensive researches based on the assumption that useful, novel membrane-type MMPs should exist other than hitherto known membrane-type MMPs that are considered useful in pharmaceutical purposes. As a result, the inventors have found novel and true MT5-MMP polypeptides and succeeded in obtaining antibodies that recognize the polypeptides. Thus, the present invention has been achieved.

The present invention relates to the following inventions (1) to (20).
(1) An antibody which recognizes a polypeptide consisting of the amino acid sequence as shown in SEQ ID NO: 1.
(2) An antibody which recognizes a polypeptide consisting of an amino acid sequence wherein one or several amino acids are deleted, substituted or added in the amino acid sequence of the polypeptide according to (1) above and having metalloproteinase activity.
(3) An antibody which recognizes a polypeptide consisting of the amino acid sequence as shown in SEQ ID NO: 2.
(4) An antibody which recognizes a polypeptide consisting of an amino acid sequence wherein one or several amino acids are deleted, substituted or added in the amino acid sequence of the polypeptide according to (3) above and having metalloproteinase activity.
(5) An antibody which recognizes a polypeptide consisting of the amino acid sequence as shown in SEQ ID NO: 5.
(6) An antibody which recognizes a polypeptide consisting of an amino acid sequence wherein one or several amino acids are deleted, substituted or added in the amino acid sequence of the polypeptide according to (5) above and having metalloproteinase activity.
(7) An antibody which recognizes a polypeptide consisting of the amino acid sequence as shown in SEQ ID NO: 6.
(8) An antibody which recognizes a polypeptide consisting of an amino acid sequence wherein one or several amino acids are deleted, substituted or added in the amino acid sequence of the polypeptide according to (7) above and having metalloproteinase activity.
(9) A method of immunological detection of the polypeptide according to any one of (1) to (8) above, which comprises using the antibody according to any one of (1) to (8) above.
(10) The method of immunological detection according to (9) above, wherein said method is selected from the group consisting of fluorescent antibody technique, enzyme-linked immunosorbent assay, radioimmunoassay, immunohistochemical staining, Western blotting, immunoprecipitation, enzyme immunoassay and sandwich ELISA.
(11) A method of immunological quantitative determination of the polypeptide according to any one of (1) to (8) above, which comprises using the antibody according to any one of (1) to (8) above.
(12) The method of immunological quantitative determination according to (11) above, wherein said method is an immunological detection method selected from the group consisting of fluorescent antibody technique, enzyme-linked immunosorbent assay, radioimmunoassay, immunohistochemical staining, Western blotting, immunoprecipitation, enzyme immunoassay and sandwich ELISA.
(13) A diagnostic agent, therapeutic agent or prophylactic agent for arthrosis deformans, rheumatoid arthritis, asthma, autoimmune diseases, atopic dermatitis, psoriasis, contact dermatitis, alopecia, ischemic heart diseases, immune response associated with organ transplantation, hepatitis, nephritis, pancreatitis, arteriosclerosis, leukemia, malignant tumor, wounds, corneal ulcer, tissue injury or inflammations associated with infiltration of leukocytes, wherein said agent comprises the antibody according to any one of (1) to (4) above.
(14) A method of screening for a compound that modulates the expression of the polypeptide according to any one of (1) to (4) above, which comprises contacting a cell expressing the polypeptide with a test sample and determining the amount of the polypeptide using the antibody according to any one of (1) to (4) above.
(15) A method of screening for a compound that binds to the polypeptide according to any one of (1) to (4) above, which comprises contacting the polypeptide with a test sample and then determining the amount of the antibody according to any one of (1) to (4) above which can bind to the polypeptide.
(16) A diagnostic agent, therapeutic agent or prophylactic agent for arthrosis deformans, rheumatoid arthritis, asthma, autoimmune diseases, atopic dermatitis, psoriasis, contact dermatitis, alopecia, ischemic heart diseases, immune response associated with organ transplantation, hepatitis, nephritis, pancreatitis, arteriosclerosis, leukemia, malignant tumor, wounds, corneal ulcer, tissue injury or inflammations associated with infiltration of leukocytes, wherein said agent comprises the compound obtained by the method according to (14) or (15) above.
(17) A diagnostic agent, therapeutic agent or prophylactic agent for arthrosis deformans, rheumatoid arthritis, asthma, autoimmune diseases, atopic dermatitis, psoriasis, contact dermatitis, alopecia, ischemic heart diseases, immune response associated with organ transplantation, hepatitis, nephritis, pancreatitis, arteriosclerosis, leukemia, malignant tumor, wounds, corneal ulcer, tissue injury, inflammations associated with infiltration of leukocytes, brain disorders at the time of cerebral apoplexy, Alzheimer's disease, dementia, multiple sclerosis, Parkinson's disease or brain tumor, wherein said agent comprises the antibody of any one of (5) to (8) above.
(18) A method of screening for a compound that modulates the expression of the polypeptide according to any one of (5) to (8) above, which comprises contacting a cell expressing the polypeptide with a test sample and determining the amount of the polypeptide using the antibody according to any one of (5) to (8) above.
(19) A method of screening for a compound that binds to the polypeptide according to any one of (5) to (8) above, which comprises contacting the polypeptide with a test sample and then determining the amount of the antibody according to any one of (5) to (8) above which binds to the polypeptide.
(20) A diagnostic agent, therapeutic agent or prophylactic agent for arthrosis deformans, rheumatoid arthritis, asthma, autoimmune diseases, atopic dermatitis, psoriasis, contact dermatitis, alopecia, ischemic heart diseases, immune response associated with organ transplantation, hepatitis, nephritis, pancreatitis, arteriosclerosis, leukemia, malignant tumor, wounds, corneal ulcer, tissue injury, inflammations associated with infiltration of leukocytes, brain disorders at the time of cerebral apoplexy, Alzheimer's disease, dementia, multiple sclerosis, Parkinson's disease or brain tumor, wherein said agent comprises the compound obtained by the method according to (18) or (19) above.

### BRIEF DESCRIPTION OF THE DRAWINGS

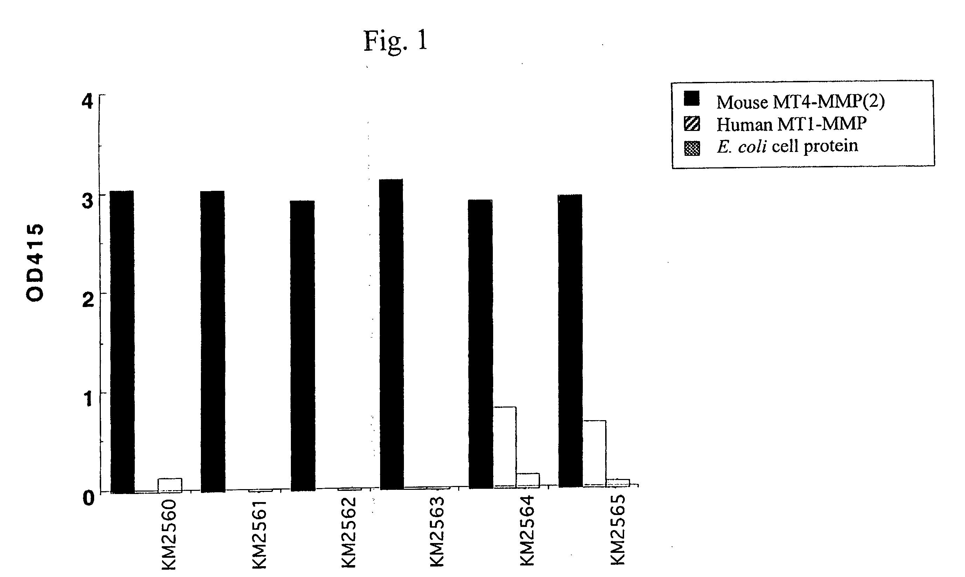

Fig. 1 shows the reaction specificities of anti-mouse MT4-MMP(2) monoclonal antibodies. The reaction specificities of KM2560-KM2565 against mouse MT4-MMP(2) hemopexin-like domain expressed by *E*. *coli* ["Mouse MT4-MMP(2)" in this Figure], human MT1-MMP hemopexin-like domain expressed by *E*. *coli* ("Human MT1-MMP" in this Figure) and an E. *coli* cell protein were examined by ELISA.

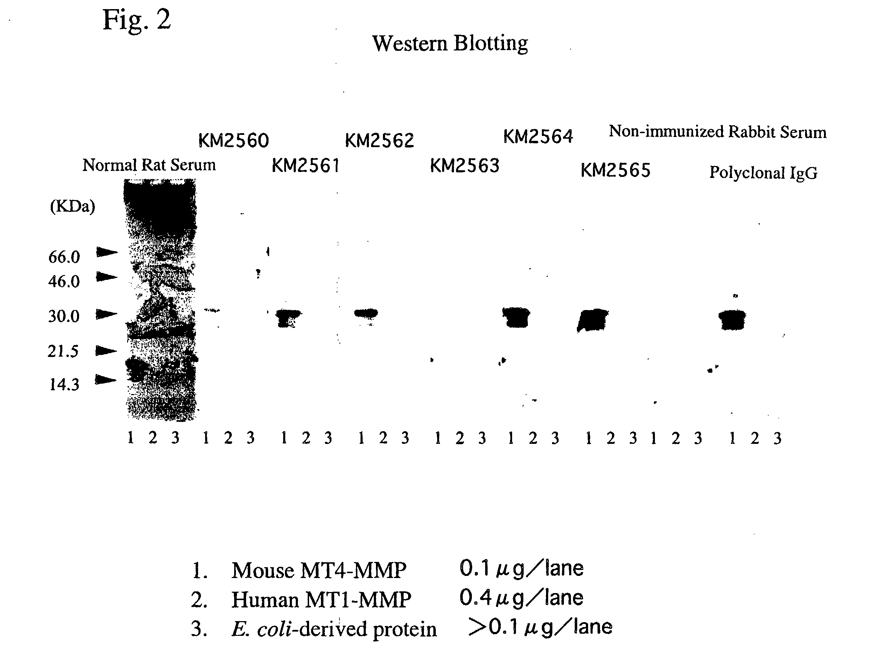

Fig. 2 shows the reaction specificities of anti-mouse MT4-MMP(2) monoclonal antibodies. The reaction specificities of KM2560-KM2565 and a rabbit polyclonal antibody (IgG fraction) against mouse MT4-MMP(2) hemopexin-like domain expressed by *E*. *coli* ("Mouse MT4-MMP" in this Figure], human MT1-MMP hemopexin-like domain expressed by E. *coli* ("Human MT1-MMP" in this Figure) and an *E*. *coli* cell protein were examined by Western blotting.

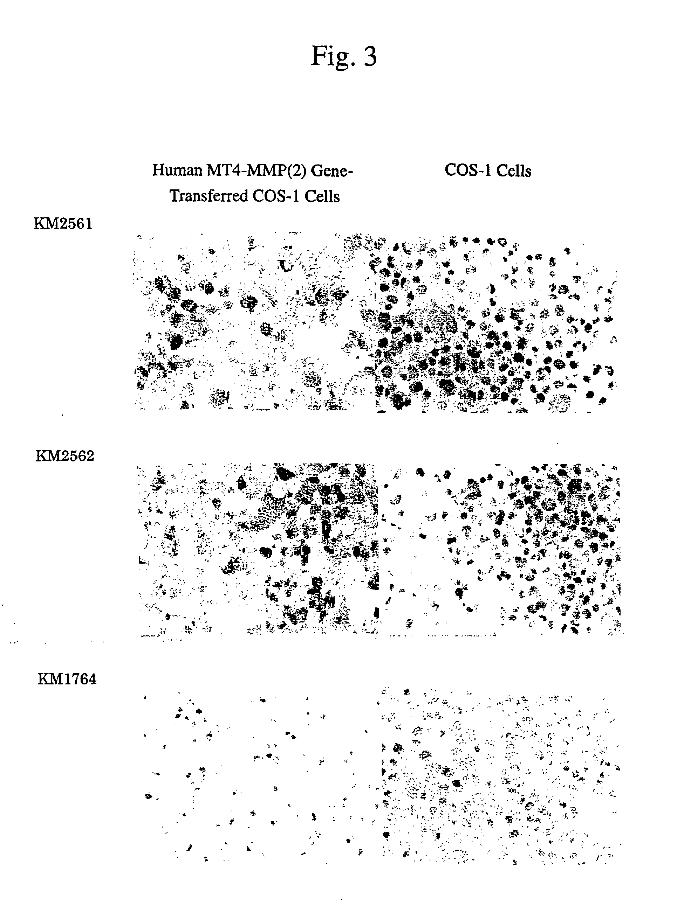

Fig. 3 shows the detection of human MT4-MMP(2)-expressing COS-1 cells by immunological staining. The reactivities of anti-mouse MT4-MMP(2) monoclonal antibodies (KM2561 and KM2562) and human MT4-MMP(2) gene-transferred COS-1 cells were examined by immunological staining. As a negative control cell, untreated COS-1 cells were used. As a negative control antibody, KM1764 (rat IgG2a) was used.

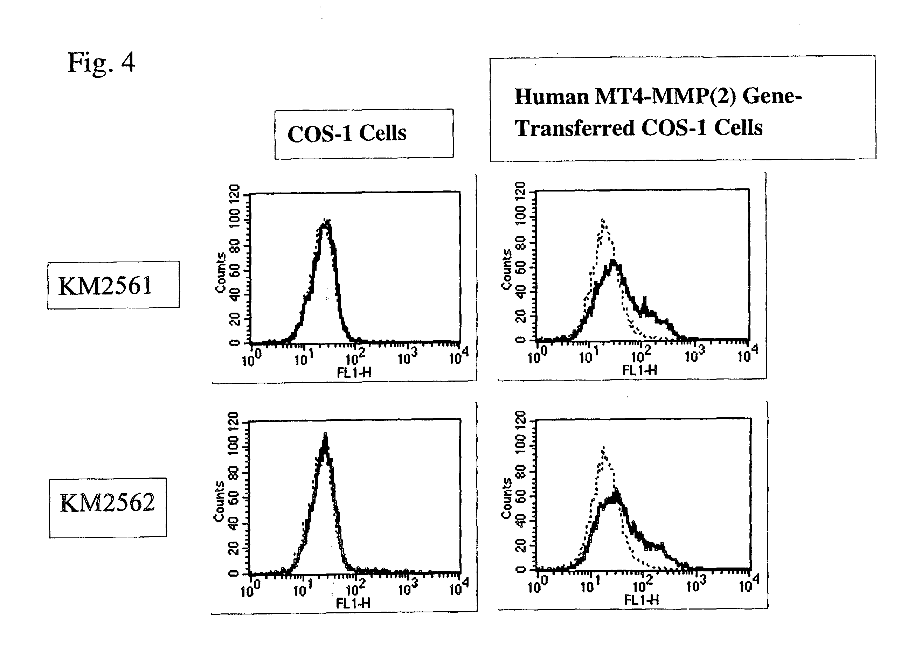

Fig. 4 shows the detection of human MT4-MMP(2) polypeptide by fluorescein antibody technique. The reactivities of anti-mouse MT4-MMP(2) monoclonal antibodies (KM2561 and KM2562) and human MT4-MMP(2) gene-transferred COS-1 cells were examined by fluorescein antibody technique. As a negative control cell, untreated COS-1 cells were used. As a negative control antibody, KM1764 (rat IgG2a) was used. The axis of ordinates represents the number of cells, and the axis of abscissas (FL1-H) represents fluorescence intensity. The dotted line represents the pattern when the control (KM1764) was added, and the solid line represents the pattern when the monoclonal antibody (KM2561 or KM2562) was added.

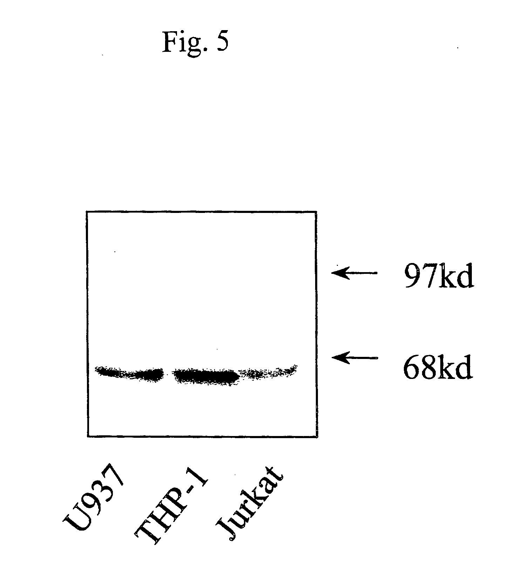

Fig. 5 shows the detection of human MT4-MMP(2) polypeptide by Western blotting. Samples (solubilized cell solutions) (100 *µ* g/lane) were subjected to SDS-PAGE (7.5% acrylamide) followed by Western blotting using anti-mouse MT4-MMP(2) monoclonal antibody KM2561. Cells used in this experiment were U937 (human histiocytic lymphoma), THP-1 (human monocyte) and Jurkat (human acute T cell leukemia), all of which were purchased from ATCC.

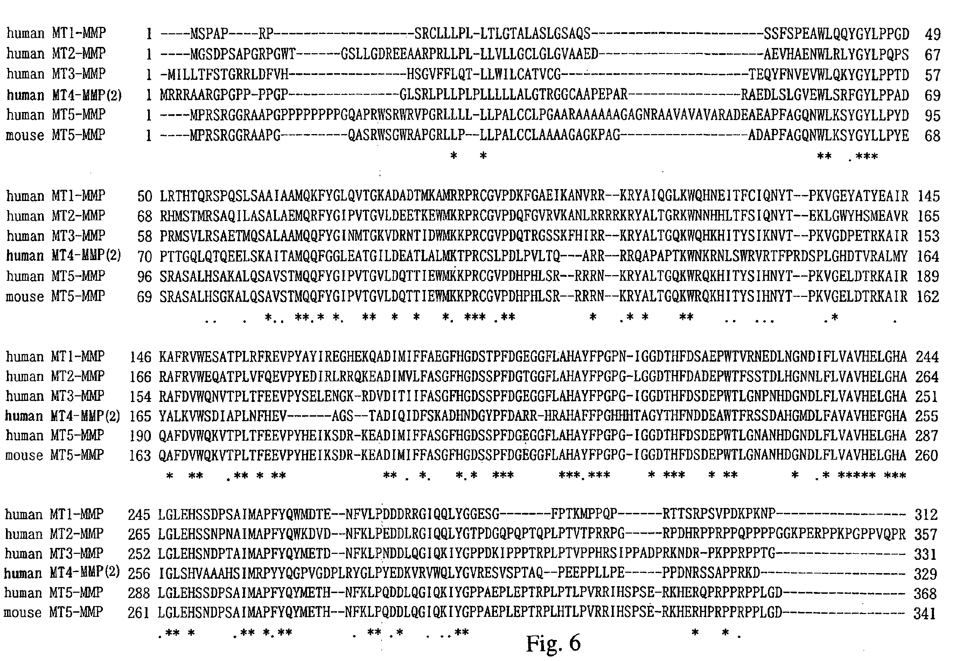

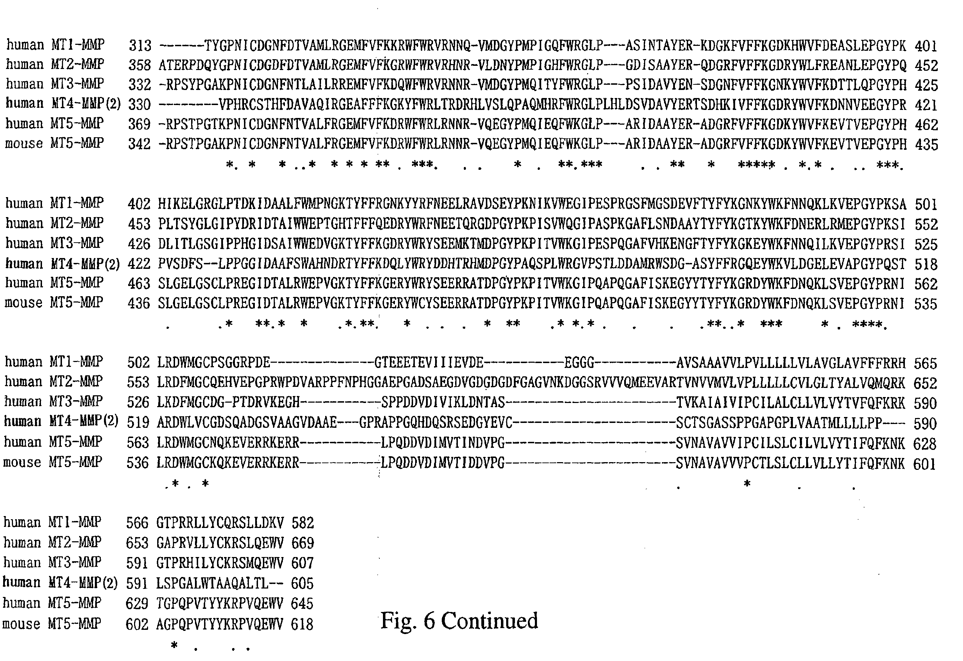

Fig. 6 shows an alignment of the amino acid sequences of human MT5-MMP and mouse MT5-MMP with the amino acid sequences of human MT1-MMP, MT2-MMP, MT3-MMP and MT4-MMP(2).

Mark "^{*}" indicates identical amino acid residues.

Mark "." indicates similar amino acid residues.
(Amino acid residues are represented by one-letter abbreviations.)
In this Figure, "kb" means kilobase pairs.

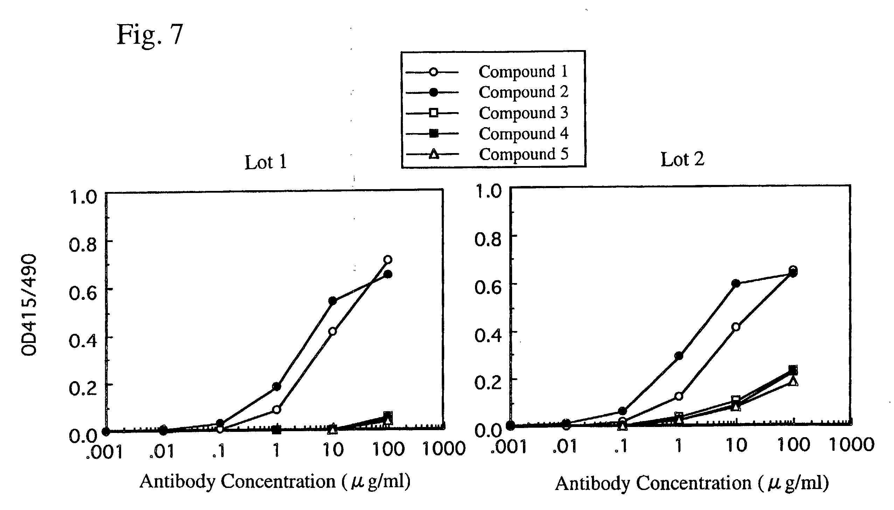

Fig. 7 shows the reaction specificity of an anti-human MT5-MMP polyclonal antibody. The reactivities of which lot 1 and lot 2 respectively react with Compounds 1, 2 and 4 (that were used as the immunogen for antibody preparation) were examined by ELISA. Compounds 3 and 5, which are not the immunogen, were used as controls.

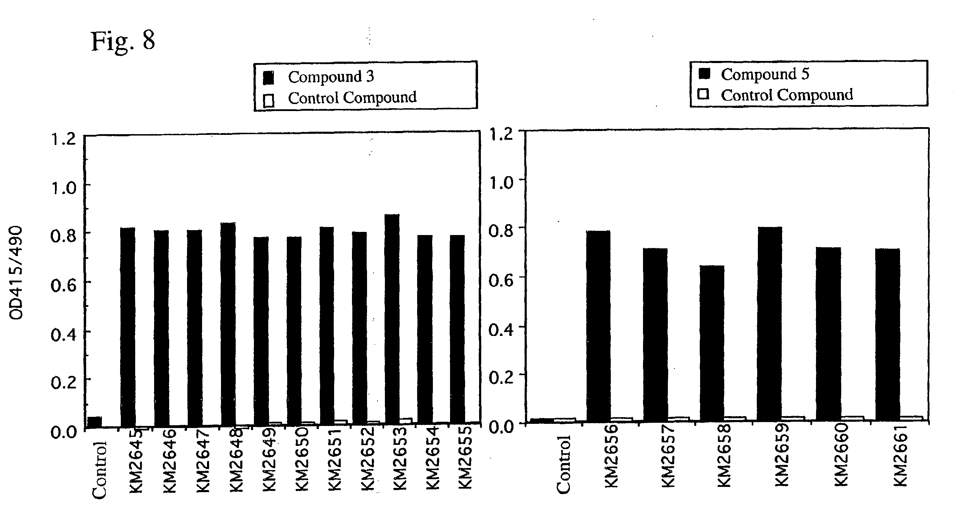

Fig. 8 shows the reaction specificities of anti-human MT5-MMP monoclonal antibodies. The reactivities of KM2645-KM2655 and the immunogen (i.e. Compound 3) and the reactivities of KM2656-KM2661 and the immunogen (i.e. Compound 5) were examined by ELISA. As a control, reaction was carried out without addition of the primary antibody. As a control compound, Compound 5 was used for KM2645-KM2655 and Compound 3 was used for KM2656-KM2661.

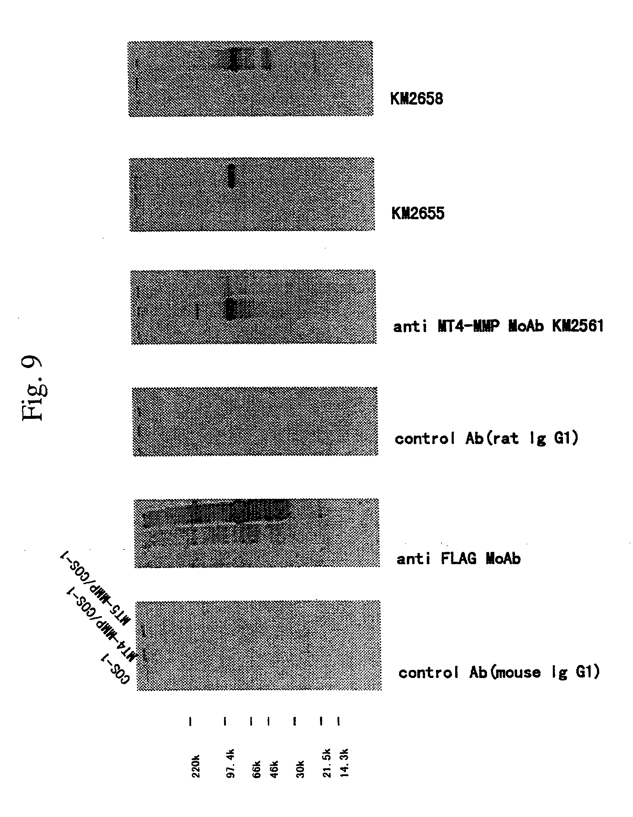

Fig. 9 shows the results of detecting human MT5-MMP polypeptide by Western blotting. A solubilized solution of COS-1 cells ("COS-1" in this Figure), a solubilized solution of human MT4-MMP(2) gene-transferred COS-1 cells ("MT4-MMP/COS-1" in this Figure), and a solubilized solution of human MT5-MMP gene-transferred COS-1 cells ("MT5-MMP/COS-1" in this Figure) were subjected to SDS-PAGE (7.5% acrylamide). Western blotting was carried out using anti-human MT5-MMP monoclonal antibodies KM2655 and KM2658. Anti-FLAG monoclonal antibody, anti-MT4-MMP monoclonal antibody KM2561, mouse IgG1 and rat IgG1 were used as control antibodies.

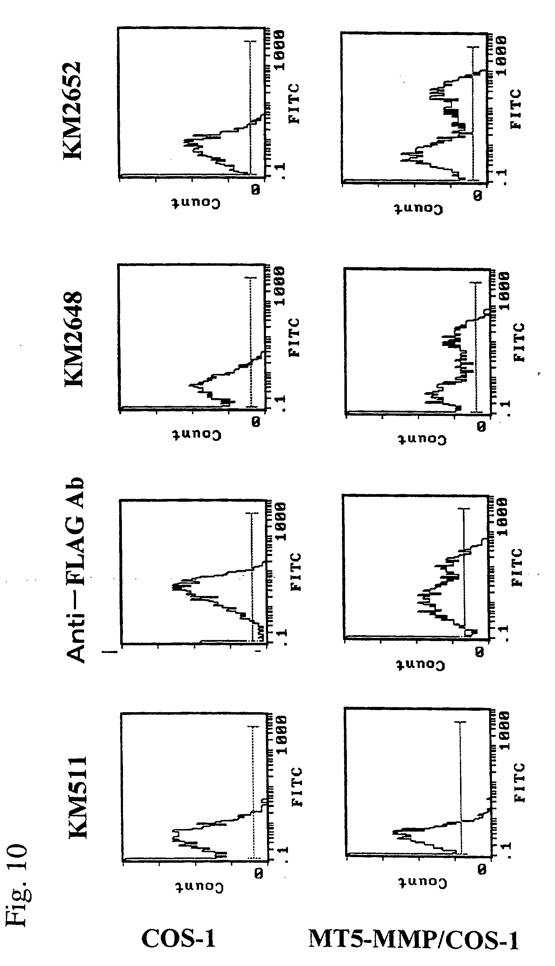

Fig. 10 shows the results of detecting human MT5-MMP polypeptide by fluorescein antibody technique. The reactivities of which anti-human MT5-MMP monoclonal antibodies (KM2648 and KM2652 obtained from Compound 3) react with human MT5-MMP gene-transferred COS-1 cells ("MT5-MMP/COS-1" in this Figure) and untreated COS-1 cells were examined by fluorescein antibody technique. The axis of ordinates represents the number of cells, and the axis of abscissas represents fluorescence intensity. As control antibodies, an anti-FLAG monoclonal antibody and a monoclonal antibody that does not react with MT5-MMP (anti-G-CSF derivative monoclonal antibody KM511) were used.

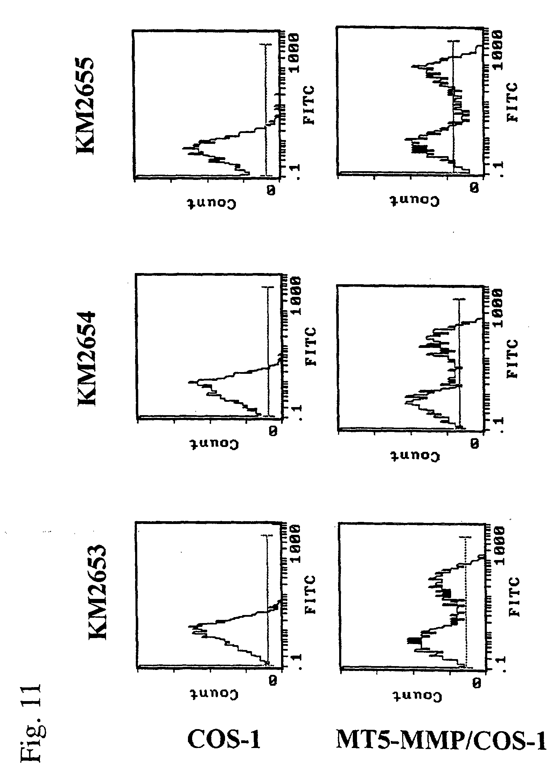

Fig. 11 shows the results of detecting human MT5-MMP polypeptide by fluorescein antibody technique. The reactivities of which anti-human MT5-MMP monoclonal antibodies (KM2653, KM2654 and KM2655 obtained from Compound 3) react with human MT5-MMP gene-transferred COS-1 cells ("MT5-MMP/COS-1" in this Figure) and untreated COS-1 cells were examined by fluorescein antibody technique. The axis of ordinates represents the number of cells, and the axis of abscissas represents fluorescence intensity.

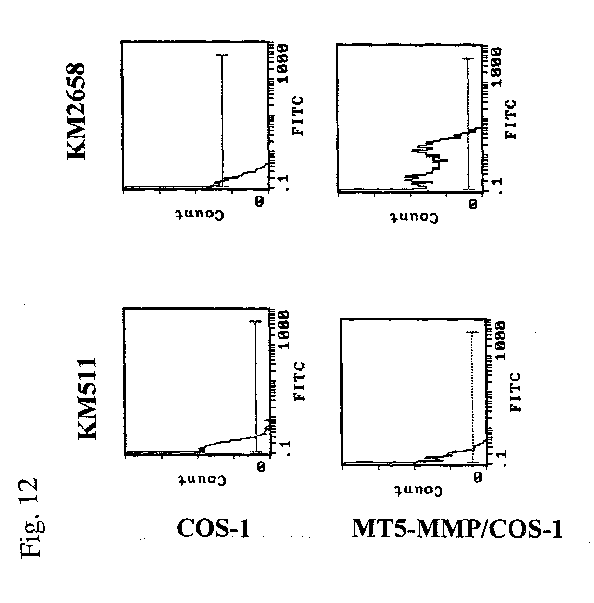

Fig. 12 shows the results of detecting human MT5-MMP polypeptide by fluorescent antibody technique. The reactivities of which an anti-human MT5-MMP monoclonal antibody (KM2658 obtained from Compound 5) reacts with human MT5-MMP gene-transferred COS-1 cells ("MT5-MMP/COS-1" in this Figure) and untreated COS-1 cells were examined by fluorescein antibody technique. The axis of ordinates represents the number of cells, and the axis of abscissas represents fluorescence intensity. As a control antibody, a monoclonal antibody that does not react with MT5-MMP (anti-G-CSF derivative monoclonal antibody KM511) was used.

### BEST MODES FOR CARRYING OUT THE INVENTION

Hereinbelow, the present invention will be described in detail.

### [1] Acquisition of DNAs encoding the Novel Matrix Metalloproteinases MT4-MMP(2) and MT5-MMP (hereinafter, referred to as "the polypeptide(s) of the present invention" unless otherwise indicated explicitly)

### (1) Preparation of cDNA Libraries

In order to construct a cDNA library, total RNA or mRNA is prepared from an appropriate cell or tissue.

As a method for preparing total RNA, the guanidine thiocyanate-cesium trifluoroacetate method [Methods in Enzymology, 154, 3 (1987)], the acid guanidine thiocyanate/phenol/chloroform (AGPC) method [Analytical Biochemistry, 162, 156 (1987); Experimental Medicine, 9, 1937 (1991)]; or the like may be used.

As a method for preparing mRNA (as poly(A)⁺ RNA) from total RNA, a method using oligo(dT) immobilized cellulose column (Molecular Cloning, A Laboratory Manual, Second Edition, Cold Spring Harbor Laboratory Press (1989); hereinafter, abbreviated to "Molecular Cloning 2nd Ed."), a method using oligo(dT) latex [Cell Engineering, Supplement 8, "New Cell Engineering Experiment Protocols", SHUJUNSHA Co., pp.48-52; Nucleic Acids Res., Symposium Series, 19, 61 (1988)] or the like may be used.

Alternatively, mRNA may be prepared directly from a tissue or cell using a commercial kit such as Fast Track mRNA Isolation Kit (Invitrogen) or Quick Prep mRNA Purification Kit (Pharmacia).

In the case of MT4-MMP(2), preferably, types of cDNA libraries which contained ESTs of the DNA encoding MT4-MMP found in databases are ascertained, and then cells or tissues that were used for the construction of those libraries, or cell lines, etc. derived from those tissues may be used as an appropriate cell or tissue.

In the case of MT5-MMP, it is preferable to use tissues such as brain and kidney or cell strains derived from those tissues as an appropriate cell or tissue.

From the resultant total RNA or mRNA, a cDNA library is constructed by conventional methods.

Examples of methods for constructing cDNA libraries include those described in Molecular Cloning 2nd Ed.; Current Protocols in Molecular Biology, John Wiley & Sons (1987-1997) (hereinafter, abbreviated to "Current Protocols in Molecular Biology"); DNA Cloning 1: Core Techniques, A Practical Approach, Second Edition, Oxford University Press (1995); etc. or methods using commercial kits such as SuperScript Plasmid System for cDNA Synthesis and Plasmid Cloning manufactured by Gibco BRL or ZAP-cDNA Synthesis Kit manufactured by Stratagene.

As a cloning vector for constructing a cDNA library, any vector, such as a phage vector or plasmid vector, may be used as long as it is capable of autonomous replication in *E*. *coli* K12 strain.

Specifically, ZAP Express [Stratagene; Strategies, 5, 58 (1992)], pBluescript II SK(+) [Nucleic Acids Research, 17, 9494 (1989)], Lamda ZAP II (Stratagene), λ gt10, λ gt11 [DNA Cloning, A Practical Approach, 1, 49 (1985)], λ TriplEx (Clontech), λ ExCell (Pharmacia), pT7T318U (Pharmacia), pcD2 [Mol. Cell. Biol., 3, 280 (1983)], pUC18 [Gene, 33, 103 (1985)], pAMo [J. Biol. Chem., 268, 22782-22787 (1993); also called as "pAMoPRC3Sc" (Japanese Unexamined Patent Publication No. 05-336963) or the like may be used.

As a host microorganism, any microorganism may be used as long as it belongs to *Escherichia coli*. Specifically, *Escherichia coli* XL1-Blue MRF' [Stratagene; Strategies 5, 81 (1992)], *Escherichia coli* C600 [Genetics, 39, 440 (1954)], *Escherichia coli* Y1088 [Science, 222, 778 (1983)], *Escherichia coli* Y1090 [Science, 222, 778 (1983)], *Escherichia coli* NM522 [J. Mol. Biol., 166, 1 (1983)], *Escherichia coli* K802 [J. Mol. Biol., 16, 118 (1966)], *Escherichia coli* JM105 [Gene, 38, 275 (1985)], *Escherichia coli* SOLRTM Strain (Stratagene), *Escherichia coli* LE392 (Molecular Cloning 2nd Ed.) or the like may be used.

In addition to cDNA libraries constructed by the above-described methods, commercial cDNA libraries may also be used.

Examples of commercial cDNA libraries include cDNA libraries of individual organs derived from animals such as human, bovine, mouse, rat or rabbit manufactured by Clontech, Lifetech Oriental, etc.

### (2) Aquisition of DNAs encoding the Polypeptides of the Invention

cDNA clones containing the DNA of the present invention may be selected From the cDNA library prepared in (I) above by such method as colony hybridization or plaque hybridization (Molecular Cloning 2nd Ed.) using a radioactively or fluorescently labeled probe.

As a probe for MT4-MMP(2) gene, an oligonucleotide based on the nucleotide sequence of a DNA encoding MT4-MMP (a part of which has been elucidated) may be used. For MT5-MMP gene, an oligonucleotide based on the nucleotide sequence of a DNA encoding MT3-MMP may be used.

From the resultant clones of interest, mRNA is obtained as described above and then cDNA is synthesized.

An adaptor is added to both ends of the resultant cDNA. Using a primer based on the sequence of this adaptor and a gene-specific primer based on the partially known sequence of the gene of interest, 5' RACE (rapid amplification of cDNA ends) and 3' RACE [Proc. Natl. Acad. Sci. USA, 85, 8998 (1988)] are carried out to obtain a cDNA fragment located 5' to the primer sequence and a cDNA fragment located 3' to the primer sequence.

By ligating the resultant cDNA fragments, a full-length cDNA can be obtained.

The nucleotide sequence of the thus obtained DNA fragment can be determined by integrating into a vector the fragment as it is or the fragment digested with an appropriate restriction enzyme by conventional methods and then analyzing the sequence by conventional methods such as the dideoxy method by Sanger et al. [Proc. Natl. Acad. Sci. USA, 74, 5463 (1977)] or with a DNA sequencer manufactured by Perkin Elmer (373A DNA Sequencer), Pharmacia, LI-COR, etc.

In order to determine the nucleotide sequence of the genomic DNA fragment encoding the polypeptide of the present invention, conventional methods for chromosomal DNA cloning (Molecular Cloning 2nd Ed.) can be used.

Briefly, chromosomal DNA from cells expressing the polypeptide of the present invention [such as monocytic THP-1 cells for MT4-MMP(2); such as brain or kidney cells for MT5-MMP] is digested with a restriction enzyme. The digested fragments are clones into a conventional plasmid vector or phage vector to construct a genomic library.

The genomic library is screened using, as a probe, the DNA fragment obtained and sequenced as described above in the same manner as in the cDNA cloning described above. Thus, clones containing the genomic gene encoding the polypeptide of the present invention can be obtained.

Using the resultant clones, the nucleotide sequence of the genomic gene can be determined by the above-described method.

It is also possible to obtain a DNA of interest derived from other tissues or other animals (e.g. human) by selecting DNAs that hybridize to the DNA obtained by the above-described method under stringent conditions.

Alternatively, a DNA of interest may be chemically synthesized with a DNA synthesizer based on the nucleotide sequence information obtained by the above-described method. As a DNA synthesizer, one using the thiophosphite method manufactured by Shimadzu Corp., a DNA synthesizer model 392 using the phosphoamidite method manufactured by Perkin Elmer, or the like may be enumerated.

The novelty of the nucleotide sequence obtained can be confirmed by searching DNA sequence databases of GenBank, EMBL, DDBJ, etc. using a homology search program such as BLAST. If the nucleotide sequence is found to be novel, it is converted into an amino acid sequence. Then, amino acid sequence databases of GenPept, PIR, Swiss-Prot, etc. are searched using a homology search program such as FASTA or FrameSearch to thereby search for existing genes having homology to the novel nucleotide sequence.

As a DNA encoding MT4-MMP(2) that has been confirmed to have a novel nucleotide sequence by the above-described method, a DNA having the nucleotide sequence as shown in SEQ ID NO: 3 or SEQ ID NO: 4 may be given, for example.

As a plasmid comprising a DNA having the nucleotide sequence as shown in SEQ ID NO: 3, plasmid pmMT4/pBSSK may be given. As a plasmid comprising a DNA having the nucleotide sequence as shown in SEQ ID NO: 4, plasmid phMT4/pBSIIKS may be given.

*Escherichia coli* pmMT4/pBSSK comprising plasmid pmMT4/pBSSK and *Escherichia coli* phMT4/pBSIIKS comprising plasmid phMT4/pBSIIKS were deposited as FERM BP-6528 and FERM BP-6530, respectively, on September 25, 1998 with National Institute of Bioscience and Human-Technology, Agency of Industrial Science and Technology located at 1-3, Higashi 1-chome, Tsukuba City, Ibaraki Pref., Japan (postal code: 305-8566).

As a DNA encoding another polypeptide of the present invention that has been confirmed to have a novel nucleotide sequence by the above-described method, a DNA having the nucleotide sequence as shown in SEQ ID NO: 7 or SEQ ID NO: 8 may be given, for example.

As a plasmid comprising a DNA having the nucleotide sequence as shown in SEQ ID NO: 7, plasmid pmMT5/pBSSK may be given. As a plasmid comprising a DNA having the nucleotide sequence as shown in SEQ ID NO: 8, plasmid phMT5/pGEM may be given.

*Escherichia coli* pmMT5/pBSSK comprising plasmid pmMT5/pBSSK and *Escherichia coli* phMT5/pGEM comprising plasmid phMT5/pGEM were deposited as FERM BP-6529 and FERM BP-6531, respectively, on September 25, 1998 with National Institute of Bioscience and Human-Technology, Agency of Industrial Science and Technology located at 1-3, Higashi 1-chome, Tsukuba City, Ibaraki Pref., Japan (postal code: 305-8566).

### [2] Preparation of the Matrix Metalloproteinase Polypeptides

### (1) Preparation of Transformants

In order to express in a host cell the DNA encoding the polypeptide of the present invention obtained by the method described in [1] above (hereinafter, referred to as "the DNA of the present invention"), methods described in Molecular Cloning 2nd Ed. and Current Protocols in Molecular Biology, for example, may be used.

Briefly, a recombinant expression vector is prepared by inserting the DNA of the present invention downstream of a promoter in an appropriate expression vector. Then, by introducing the recombinant vector into a host cell, a transformant that expresses the polypeptide of the present invention can be obtained.

As a host cell, any cell such as a bacterium, yeast, animal cell, or insect cell may be used as long as it is capable of expressing the gene of interest.

As an expression vector, a vector that is capable of autonomous replication in the host cell or capable of being integrated into chromosomes of thereof and that contains a promoter at a site appropriate for transcription of the DNA of the present invention is used.

When a procaryote such as a bacterium is used as the host cell, it is preferred that the expression vector for the polypeptide gene of the present invention be capable of autonomous replication in the procaryote and, at the same time, a recombinant vector composed of a promoter, a ribosome binding sequence, the DNA of the present invention, and a transcription termination sequence. The vector may also contain a gene that controls the promoter.

Examples of expression vectors which may be used in the present invention include pKK233-2 (Pharmacia), pSE280 (Invitrogen), pGEMEX-1 (Promega), pQE-8 (Qiagen), pKYP10 (Japanese Unexamined Patent Publication No. 58-110600), pKYP200 [Agric. Biol. Chem., 48, 669 (1984)], pLSA1 [Agric. Biol. Chem., 53, 277 (1989)], pGEL1 [Proc. Natl. Acad. Sci. USA, 82, 4306 (1985)], pBluescript II SK(-) (Stratagene), pGEX (Pharmacia), and pET-3 (Novagen).

As a promoter, any promoter may be used as long as it can direct the expression of the gene of interest in a host cell such as *E*. *coli* or *Bacillus subtilis.* For example, an *E. coli-* or phage-derived promoter such as trp promoter (Ptrp), lac promoter, PL promoter, PR promoter or T7 promoter; SP01 promoter; SP02 promoter; or penP promoter may be used. An artificially designed and altered promoter such as a promoter in which two Prtp promoters are connected in series (Ptrp x 2), tac promoter, lacT7 promoter, or let I promoter may also be used.

As a ribosome binding sequence, it is preferable to use a plasmid in which the distance between Shine-Dalgarno sequence and the initiation codon is appropriately adjusted (e.g., 6-18 bp).

In the recombinant vector of the present invention, it is not necessarily required for the expression of the DNA of the present invention to contain a transcription termination sequence, but it is desirable to locate such a sequence immediately downstream of the structural gene.

As a host cell, a microorganism belonging to the genus *Escherichia*, *Serratia*, *Bacillus*, *Brevibacterium*, *Corynebacterium*, *Microbacterium*, *Pseudomonas* or the like may be used. Examples of host cells which may be used in the present invention include *Escherichia coli* XL1-Blue, *Escherichia coli* XL2-Blue, *Escherichia coli* DH1, *Escherichia coli* MC1000, *Escherichia coli* KY3276, *Escherichia coli* W1485, *Escherichia coli* JM109, *Escherichia coli* HB101, *Escherichia coli* No.49, *Escherichia coli* W3110, *Escherichia coli* NY49, *Serratia ficaria*, *Serratia fonticola*, *Serratia liquefaciens*, *Serratia marcescens, Bacillus subtilis*, *Bacillus amyloliquefaciens*, *Brevibacterium ammmoniagenes*, *Brevibacterium immariophilum* ATCC14068, *Brevibacterium saccharolyticum* ATCC14066, *Corynebacterium glutamicum* ATCC13032, *Corynebacterium glutamicum* ATCC14067, *Corynebacterium glutamicum* ATCC13869, *Corynebacterium acetoacidophilum* ATCC13870, *Microbacterium ammoniaphilum* ATCC15354, and *Pseudomonas* sp. D-0110.

As a method for introducing the recombinant vector, any method of introducing DNA into the above host cell may be used. For example, the method using calcium ions [Proc. Natl. Acad. Sci., USA, 69, 2110 (1972)], the protoplast method (Japanese Unexamined Patent Publication No. 63-248394), or electroporation [Gene, 17, 107 (1982); Molecular & General Genetics, 168, 111 (1979)] maybe used.

When a yeast strain is used as the host cell, an expression vector such as YEp13 (ATCC37115), YEp24 (ATCC37051), YCp50 (ATCC37419), pHS19, or pHS15 may be used.

As a promoter, any promoter that can direct the expression of the gene of interest in yeast may be used. For example, PH05 promoter, PGK promoter, GAP promoter, ADH promoter, gal 1 promoter, gal 10 promoter, heat shock polypeptide promoter, MF α 1 promoter, or CUP 1 promoter may be used.

As a host cell, a yeast strain belonging to the genus *Saccharomyces, Schizosaccharomyces*, *Kluyveromyces*, *Trichosporon*, *Schwanniomyces*, *Pichia* or the like may be used. Examples of yeast strains that may be used in the present invention include *Saccharomyces cerevisiae*, *Schizosaccharomyces pombe*, *Kluyveromyces lactis, Trichosporon pullulans, Schwanniomyces alluvius*, and *Pichia pastoris.*

As a method for introducing the recombinant vector, any method of introducing DNA into yeast may be used. For example, electroporation [Methods in Enzymology, 194, 182 (1990)], the spheroplast method [Proc. Natl. Acad. Sci., USA, 81, 4889 (1984)], the lithium acetate method [Journal of Bacteriology, 153, 163 (1983)] or the like may be enumerated.

When an animal cell is used as the host, an expression vector such as pAGE107 (Japanese Unexamined Patent Publication No. 3-22979; Cytotechnology, 3, 133 (1990)], pAS3-3 (Japanese Unexamined Patent Publication No. 2-227075), pCDM8 [Nature, 329, 840 (1987)], pcDNAI/Amp (Invitrogen), pREP4 (Invitrogen), or pAGE103 [Journal of Biochemistry, 101, 1307 (1987)] may be used.

As a promoter, any promoter that can direct the expression of the gene of interest in animal cells may be used. Examples of promoters that may be used in the present invention include the promoter of the IE (immediate early) gene of cytomegalovirus (CMV), the early promoter of SV40, a metallothionein promoter, a retrovirus promoter, a heat shock promoter and SR α promoter. Alternatively, the enhancer of the IE gene of human CMV may be used in combination with the promoter thereof.

Examples of animal cells that may be used in the present invention include human cells such as Namalwa cells, HEK293 cells (ATCC: CRL-1573); simian cells such as COS cells; and Chinese hamster cells such as CHO cells, HBT5637 (Japanese Unexamined Patent Publication No. 63-299).

As a method for introducing the recombinant vector, any method of introducing DNA into animal cells may be used. For example, electroporation [Cytotechnology, 3, 133 (1990)], the calcium phosphate method (Japanese Unexamined Patent Publication No. 2-227075), or lipofection [Proc. Natl. Acad. Sci. USA, 84, 7413 (1987); Virology, 52, 456 (1973)] may be used.

When an insect cell is used as the host, it is possible to express the polypeptide of the present invention according to methods described in, for example, Baculovirus Expression Vectors, A Laboratory Manual, W.H. Freeman and Company, New York (1992); Current Protocols 1-38; and Bio Technology, 6, 47 (1988).

Briefly, a recombinant gene transfer vector and Baculovirus are co-introduced into an insect cell to thereby obtain a recombinant virus in the supernatant of the insect cell culture. Then, the insect cell is infected with the recombinant virus further to allow the production of the polypeptide of the present invention.

As a gene transfer vector that may be used in the above method, pVL1392, pVL1393 or pBlueBacIII (all of which are manufactured by Invitrogen) may be enumerated, for example.

As a Baculovirus that may be used in the above method, *Autographa californica* nuclear polyhedrosis virus that infects insects belonging to the subfamily Hadeninae may be given, for example.

As an insect cell that may be used in the above method, *Spodoptera frugiperda* ovary cells Sf9 and Sf21 [Baculovirus Expression Vectors, A Laboratory Manual (1992)]; a *Trichoplusia ni* ovary cell High5 (Invitrogen); or the like may be enumerated.

As a method of co-introducing a gene transfer vector and Baculovirus into an insect cell for preparing a recombinant virus, the calcium phosphate method (Japanese Unexamined Patent Publication No. 2-227075) or lipofection [Proc. Natl. Acad. Sci. USA, 84, 7413 (1987)] may be enumerated, for example.

As a method of expressing the gene, in addition to direct expression, such as secretion production or fusion protein expression may be carried out based on the methods described in Molecular Cloning 2nd Ed.

When the polypeptide of the present invention is expressed by a yeast strain, animal cell or insect cell, a polypeptide to which sugars or sugar chains have been attached can be obtained.

The polypeptide of the present invention can be prepared by culturing the transformant obtained as described above in a medium, allowing the polypeptide of the present invention to be produced and accumulated in the culture, and recovering the polypeptide from the culture.

### (2) Culturing of Transformants

The culturing of the transformant of the present invention in a medium is carried out by conventional methods used for culturing hosts.

As a medium to culture the transformant obtained from a procaryotic host such as *E. coli* or an eucaryotic host such as yeast, either a natural or synthetic medium may be used as long as it contains carbon sources, nitrogen sources and inorganic salts assimilable by the microorganism and is suitable for efficient culturing of the transformant.

As carbon sources, any carbon source may be used as long as it is assimilable by the microorganism. For example, carbohydrates such as glucose, fructose, sucrose, or molasses, starch or starch hydrolysate containing them; organic acids such as acetic acid, propionic acid; and alcohols such as ethanol and propanol may be used.

As nitrogen sources, ammonia; ammonium salts of inorganic or organic acids such as ammonium chloride, ammonium sulfate, ammonium acetate, ammonium phosphate; other nitrogen-containing compounds; Peptone; meat extract; corn steep liquor; casein hydrolysate; soybean meal and soybean meal hydrolysate; various fermented microorganism cells and digested products thereof; and the like may be used.

As inorganic substances, potassium dihydrogen phosphate, dipotassium hydrogen phosphate, magnesium phosphate, magnesium sulfate, sodium chloride, iron(II) sulfate, manganese sulfate, copper sulfate, calcium carbonate and the like may be used.

Usually, the culturing is carried out under aerobic conditions, by such as shaking culture or submerged aeration agitation culture. The culturing temperature is preferably between 15 to 40°C, and the culturing period is usually 16 to 96 hrs. During the culturing, the pH is maintained at 3.0 to 9.0. The pH adjustment is carried out using an inorganic or organic salt, an alkali solution, urea, calcium carbonate, ammonia or the like.

During the culturing, an antibiotic such as ampicillin or tetracycline may be added to the medium if necessary.

When a microorganism transformed with an expression vector using an inducible promoter is cultured, an inducer may be added to the medium if necessary. For example, when a microorganism transformed with an expression vector using Lac promoter is cultured, isopropyl- β-D-thiogalactopyranoside or the like may be added. When a microorganism transformed with an expression vector using trp promoter is cultured, indoleacrylic acid or the like may be added.

As a medium to culture a transformant obtained from an animal cell as a host, commonly used RPMI1640 medium [The Journal of the American Medical Association, 199, 519 (1967)], Eagle's MEM medium [Science, 122, 501 (1952)], DMEM medium [Virology, 8, 396 (1959)], 199 medium [Proceeding of the Society for the Biological Medicine, 73, 1 (1950)] or one of these media supplemented with fetal bovine serum, etc. may be used.

Usually, the culturing is carried out at pH 6-8, at 30-40°C in the presence 5% CO₂ for 1 to 7 days.

During the culturing, an antibiotic such as kanamycin, penicillin, or streptomycin may be added to the medium if necessary.

As a medium to culture a transformant obtained from an insect cell as a host, commonly used TNM-FH medium (Pharmingen), Sf-900 II SFM medium (Life Technologies), ExCell400 or ExCell405 (both from JRH Biosciences), Grace's Insect Medium [Nature, 195, 788 (1962)] or the like may be used.

Usually, the culturing is carried out at pH 6-7 at 25-30°C for 1 to 5 days.

During the culturing, an antibiotic such as gentamycin may be added to the medium if necessary.

### (3) Isolation and Purification of the Expressed Polypeptides

Conventional methods of enzyme isolation/purification may be used to isolate and purify the polypeptides of the present invention expressed by the method described above from the culture of the above-described transformant. For example, when the polypeptide of the present invention is expressed in a state dissolved in cells, the cells are harvested by centrifugation after completion of the culturing, and then suspended in an aqueous buffer. Subsequently, the cells are disrupted with a sonicator, French press, Manton-Gaulin homogenizer, Dynomill or the like to thereby obtain a cell-free extract, which is then centrifuged to obtain a supernatant. From this supernatant, a purified sample may be obtained by conventional enzyme isolation/purification methods. For example, the solvent extraction method; salting out with ammonium sulfate or the like; desalting; precipitation with organic solvents; anion exchange chromatography using resins such as Q-Sepharose, diethylaminoethyl (DEAE)-Sepharose, DIAION HPA-75 (Mitsubishi Chemical Corp.); cation exchange chromatography using resins such as S-Sepharose FF (Pharmacia); hydrophobic chromatography using resins such as butyl Sepharose, phenyl Sepharose; gel filtration using molecular sieve; affinity chromatography; and electrophoresis such as chromatofocusing, isoelectric focusing; may be used independently or in combination.

When the polypeptide of the present invention is expressed in an insoluble form within cells, the cells are harvested and disrupted in the same manner as described above. Then, the cells are centrifuged to obtain the precipitate fraction, from which the polypeptide is recovered by conventional methods. Subsequently, the polypeptide in an insoluble form is solubilized with a protein-denaturing agent. The resultant solubilized solution is diluted until the solution no longer contains the denaturing agent or the concentration of the denaturing agent becomes so low that no protein denaturation would occur; or the solubilized solution is dialyzed. Thus, the normal steric structure of the polypeptide is restored. Subsequently, a purified sample can be obtained by using the isolation/purification methods described above.

When the polypeptide of the present invention or a derivative thereof (such as sugar-modified polypeptide) is secreted out of cells, the polypeptide or the derivative can be recovered from the culture supernatant. Briefly, the culture is treated by centrifugation, etc. in the same manner as described above to obtain the soluble fraction. From this soluble fraction, a purified sample can be obtained by using the isolation/purification methods described above. When the polypeptide of the present invention or a derivative thereof (such as sugar-modified polypeptide) is expressed on cell surfaces, the membrane fraction of the cultured cells is dissolved with a surfactant to obtain the soluble fraction. From this soluble fraction, a purified sample can be obtained by using the isolation/purification methods described above.

Alternatively, the polypeptide of the present invention may be prepared by chemical synthesis methods such as the Fmoc (fluorenylmethyloxycarbonyl) method and the tBoc (t-butyloxycarbonyl) method. The polypeptide of the present invention may also be chemically synthesized with peptide synthesizers manufactured by Advanced ChemTech, Perkin Elmer, Pharmacia, Protein Technology Instrument, Synthecell-Vega, PerSeptive, Shimadzu Corp. and so forth.

### [3] Preparation of Antibodies that recognize the Polypeptides of the Invention

### (1) Preparation of Partial Peptides for Use as Antigen

An expression vector comprising a cDNA encoding the polypeptide of the present invention is introduced into a host such as *E*. *coli,* yeast, an insect cell or an animal cell to thereby obtain a recombinant protein. When the polypeptide is human MT5-MMP protein, this protein may be purified from cultured human tumor cells or the like. Alternatively, peptides having a partial sequence of human MT4-MMP(2) or human MT5-MMP protein may be obtained by peptide synthesis.

As a partial peptide for use as antigen, a protein partial sequence consisting of 5 to 30 residues is selected. In order to obtain antibodies that recognize proteins having a non-denatured, natural structure, it is necessary to select partial sequences present on the surfaces of proteins in their steric structures should be selected as antigen peptides. Those portions present on the surfaces of proteins in their steric structures may be presumed by predicting highly hydrophilic partial sequences by the method of, for example, Kyte and Doolittle [Journal of Molecular Biology, 157, 105-132 (1982)].

Briefly, in general, there is a tendency that portions with low hydrophilicity are present inside of proteins in their steric structures and portions with high hydrophilicity on the surfaces of proteins. Also, there is a tendency that the N-terminal and C-terminal of proteins are present on the surfaces thereof. However, partial peptides thus selected are not necessarily appropriate antigen to yield intended antibodies.

Cysteine is added to the ends of partial peptides so that they are cross-linked to proteins. When partial sequences inside the proteins are selected, the peptides are acetylated at the N-terminal and amidated at the C-terminal, if necessary.

Partial peptides may be synthesized by commonly used liquid phase or solid phase peptide synthesis techniques, or an appropriate combination of these techniques, or methods based on these techniques [The Peptides, Analysis, Synthesis, Biology, vol. 1, Erhard Gross and Johannes Meinhofer (Eds.), Academic Press (1979), vol. 2 (1980), vol. 3 (1981); Nobuo Izumiya et al., Basics and Experiments in Peptide Synthesis, Maruzen Co., Ltd., 1985; Development of Pharmaceuticals (2nd Series), vol. 14, Peptide Synthesis, Haruaki Yajima (chief editor), Hirokawa Shoten Co., 1991; International Journal of Peptide and Protein Research, 35, 161 (1990)].

Alternatively, an automated peptide synthesizer may be used. Peptide synthesis using a peptide synthesizer may be carried out on a commercial peptide synthesizer manufactured by such as Shimadzu Corp., Applied Biosystems, Inc. (hereinafter, abbreviated to ABI), or Advanced ChemTech Inc., USA (hereinafter, abbreviated to ACT), using side-chain protected N^{α}-Fmoc-amino acids or N^{α}-Boc-amino acids, etc. in accordance with the synthesis program provided by each manufacturer.

Protected amino acids and support resins that are raw materials for preparing partial peptide are available from ABI, Shimadzu Corp., Kokusan Chemical, Nova Biochem, Watanabe Chemical, ACT, Peptide Institute, Inc., Ana Spec or the like. Alternatively, protected amino acids, protected organic acids or protected organic amines which are raw materials for preparing partial peptides may be synthesized according to reported methods or based on those methods [The Peptides, Analysis, Synthesis, Biology, vol. 1, Erhard Gross and Johannes Meinhofer (Eds.), Academic Press (1979), vol. 2 (1980), vol. 3 (1981); Nobuo Izumiya et al., Basics and Experiments in Peptide Synthesis, Maruzen Co., Ltd., 1985; Development of Pharmaceuticals (2nd Series), vol. 14, Peptide Synthesis, Haruaki Yajima (chief editor), Hirokawa Shoten Co., 1991; International Journal of Peptide and Protein Research, 35, 161 (1990)].

Physicochemical properties of the partial peptides can be measured with the following instruments:
Mass spectrometry analysis is performed with a mass spectrometer JMS-HX110A (JEOL Ltd.) using the FAB method.
Amino acid analysis is performed by the method of B.A. Bidlingmeyer et al. [Journal of Chromatography, 336, 93 (1984)].
Hydrolysis is performed at 110°C for 22 hr in hydrochloric acid vapor. The amino acid composition of hydrolysate is analyzed with a Waters Accq Taq amino acid analyzer.

### (2) Preparation of Polyclonal Antibodies

Polyclonal antibodies may be prepared by administering to animals purified full-length or partial fragments of the polypeptides of the present invention prepared by the method described in [2] or in (1) in [3] above, or partial peptides prepared by the method described in (1) in [3] above as antigen.

Examples of animals which may be used in the antibody preparation include rabbits, goats, 3- to 20-week old rats, mice and hamsters.

The dose of the antigen is preferably 50-100 *µ* g per animal.

When peptides are used as antigen, it is desirable to use them after covalently binding them to a carrier protein such as keyhole limpet haemocyanin or bovine thyroglobulin.

After the initial administration of the antigen, 3 to 10 times of administration is performed at intervals of I to 2 weeks. Three to seven days after each administration, blood samples are taken from the venous plexus of the eyeground to prepare sera. Then, confirmation is made that these sera react with the antigen used in the immunization, by enzyme immunoassay [Enzyme Immunoassay (ELISA), Igaku-Shoin Co., 1976; Antibodies - A Laboratory Manual, Cold Spring Harbor Laboratory Press (1988)] or the like.

Sera are collected from those non-human mammals whose sera exhibited a sufficient antibody titer against the antigen used in the immunization. Then, polyclonal antibodies can be obtained by separating and purifying these sera.

Examples of methods for separating and purifying antibodies include centrifugation, salting out using 40-50% saturated ammonium sulfate, caprylic acid precipitation [Antibodies, A Laboratory Manual, Cold Spring Harbor Laboratory (1988)], and chromatography using DEAE-Sepharose column, anion exchange column, protein A or G column, or gel filtration column; these methods may be used independently or in a combination.

### (3) Preparation of Monoclonal Antibodies

### (3-1) Preparation of Antibody-Producing Cells

The rats, mice, hamsters or the like whose sera exhibits a sufficient antibody titer against the antigen used in the immunization in (2) above are supplied as sources of antibody-producing cells.

Three to seven days after the final administration of the antigen substance to those animals that exhibits a sufficient antibody titer, the spleen is removed from them.

The spleen is cut into pieces in MEM medium (Nissui Pharmaceutical), loosened with tweezers and centrifuged at 1,200 rpm for 5 min. The supernatant is discarded.

Spleen cells in the precipitate fraction are treated with Tris-ammonium chloride buffer (pH 7.65) for 1 to 2 min to remove erythrocytes. Then, the cells are washed with MEM medium 3 times. The resultant spleen cells are used as antibody-producing cells.

### (3-2) Preparation of Myeloma Cells

As myeloma cells, an established cell line derived from mouse or rat is used. Examples of such cell strains include 8-azaguanine resistant mouse (derived from BALB/c) myeloma cell strains P3-X63Ag8-U1 (P3-U1) [Curr. Topics Microbiol. Immunol., 81, 1 (1978); Eur. J. Immunol., 6, 511 (1976)], SP2/0-Ag14 (SP-2) [Nature, 276, 269 (1978)], P3-X63-Ag8653 (653) [J. Immunol., 123, 1548 (1979)] and P3-X63-Ag8 (X63) [Nature, 256, 495 (1975)]. These cell lines are sub-cultured in 8-azaguanine medium [glutamine (1.5 mmol/L), 2-mercaptoethanol (5x10⁻⁵M), gentamycin (10 *µ* g/mL) and fetal bovine serum (FCS) (CSL; 10%)-supplemented RPMI-1640 medium (hereinafter referred to as "normal medium") to which 8-azaguanine (15 *µ* g/mL) is added further]. Three to four days before cell fusion, they are transferred into normal medium. For cell fusion, 2x10⁷ or more of these cells are used.

### (3-3) Preparation of Hybridomas

The antibody-producing cells obtained in (3-1) above and the myeloma cells obtained in (3-2) above are washed thoroughly with MEM medium or PBS (1.83 g of disodium hydrogen phosphate; 0.21 g of potassium dihydrogen phosphate; 7.65 g of sodium chloride; 1 L of distilled water; pH 7.2) and mixed to give a ratio of antibody-producing cells:myeloma cells = 5-10:1. The mixture is centrifuged at 1,200 rpm for 5 min, and the supernatant is discarded.

Cell pellet in the resultant precipitate fraction is loosened thoroughly. Then, a liquid mixture composed of 2 g of polyethylene glycol-1000 (PEG-1000), 2 mL of MEM medium and 0.7 mL of dimethylsulfoxide (DMSO) is added to the cell pellet in an amount of 0.2-1 mL per 10⁸ antibody-producing cells at 37°C while stirring. To the resultant suspension, 1-2 mL of MEM medium is added every one to two minutes (several times in the total).

Then, MEM medium is added thereto to make the total volume 50 mL.

The resultant preparation is centrifuged at 900 rpm for 5 min, and the supernatant is discarded.

Cells in the precipitate fraction are loosened gently and then suspended gently in 100 mL of HAT medium [a medium obtained by adding hypoxanthine (10⁻⁴ mol/L), thymidine (1.5x10⁻⁵ mol/L) and aminopterin (4x10⁻⁷ mol/L) to normal medium] by pipetting with a measuring pipette.

The resultant suspension is added to 96-well culture plates (100 *µ* L/well) and cultured in a 5% CO₂ incubator at 37°C for 7-14 days.

After the culturing, a part of the culture supernatant is taken and examined by the enzyme immunoassay described in, for example, Antibodies - A Laboratory Manual [Cold Spring Harbor Laboratory Press, Chapter 14 (1988)] to thereby select those hybridomas that specifically react with the polypeptide of the present invention.

As a specific example of enzyme immunoassay, the following method may be given.

An appropriate plate is coated with the purified, full-length or partial fragment sample of the polypeptide of the present invention that was used in the immunization as the antigen. Subsequently, a culture supernatant of the hybridoma or a purified antibody obtained in (3-4) below is reacted with the polypeptide sample as a primary antibody. Then, as a secondary antibody, an anti-rat immunoglobulin antibody labeled with biotin, an enzyme, a chemiluminescent substance or a radioactive compound is reacted. Then, depending on the labeling substance used, an appropriate reaction is carried out. Thus, those hybridomas that specifically react with the polypeptide of the present invention are selected as hybridomas producing monoclonal antibodies to the polypeptide of the present invention.

The selected hybridomas are subjected to cloning by limiting dilution technique twice [fist time, HT medium (HAT medium minus aminopretin) is used, and the second time, normal medium is used]. Thus, those clones that exhibit a high antibody titer stably are selected as hybridoma clones producing monoclonal antibodies to the polypeptide of the present invention.

### (3-4) Preparation of Monoclonal Antibodies

The hybridoma cells producing monoclonal antibodies to the polypeptide of the present invention obtained in (3-3) above are administered to pristane-treated (i.e., intraperitoneal administration of 0.5 mL of 2,6,10,14-tetramethylpentadecane followed by 2-week breeding) 8- to 10-week old mice or nude mice by intraperitoneal injection (5-20 x 10⁶ cells/mouse). In 10 to 21 days, the hybridoma cells are changed into abdominal dropsy cancer.

The abdominal dropsy is collected from mice whose abdominal dropsy has cancerated, and centrifuged at 3,000 rpm for 5 min to remove solid matter.

From the resultant supernatant, monoclonal antibodies may be purified and obtained in the same manner as used in the purification of polyclonal antibodies. Alternatively, monoclonal antibodies may also be purified and obtained from culture supernatants of hybridoma clones in the same manner.

The determination of classes and subclasses of antibodies is carried out using a kit such as mouse monoclonal antibody typing kit or rat monoclonal antibody typing kit. The amount of protein is determined by Lowry method or calculated from the absorbance at 280 nm.

Antibody class means isotypes of antibody. In human, there are five classes of IgG, IgA, IgM, IgD and IgE. Subclass means isotypes within class. In mouse, there are four subclasses of IgG1, IgG2a, IgG2b and IgG3. In human, there are four subclasses of IgG1, IgG2, IgG3 and IgG4.

### [4] Use of the Antibodies of the Invention

### (1) Immunological Detection and Quantitative Determination of the Polypeptides of the Invention using the Antibodies of the Invention

The polypeptides of the present invention may be detected and quantitatively determined immunologically by the methods described below using the antibodies of the present invention.

Examples of methods which may be used for these purposes include fluorescent antibody technique, enzyme-linked immunosorbent assay (ELISA), radioimmunoassay (RIA), immunohistochemical staining such as immunological tissue staining or immunological cell staining (ABC technique, CSA technique, etc.), Western blotting, immunoprecipitation, the enzyme immunosorbent assay described above, and sandwich ELISA [Monoclonal Antibody Experiment Manual, Kodansha Scientific (1987); (2nd Series) Biochemistry Experiment Course No. 5: Methods for Immuno-Biochemical Researches, Tokyo Kagaku Dojin, Co. (1986)].

Fluorescent antibody technique is a technique in which a sample such as separated cells or liquid containing disrupted cells, separated tissue or liquid containing disrupted tissue, cell culture supernatant, serum, pleural effusion, abdominal dropsy or ocular fluid is reacted with the monoclonal antibody of the present invention and then reacted with an anti-immunoglobulin antibody or a binding fragment thereof labeled with a fluorescent substance such as fluorescein isothiocyanate (FITC), followed by measurement of the fluorescent with a flow cytometer.

Enzyme-linked immunosorbent assay (ELISA) is a technique in which a sample such as separated cells or liquid containing disrupted cells, separated tissue or liquid containing disrupted tissue, cell culture supernatant, serum, pleural effusion, abdominal dropsy or ocular fluid is reacted with the monoclonal antibody of the present invention and then reacted with an anti-immunoglobulin antibody or a binding fragment thereof labeled with an enzyme such as peroxidase or biotin, followed by measurement of the developed color with an absorptiometer.

Radioimmunoassay (RIA) is a technique in which a sample such as separated cells or liquid containing disrupted cells, separated tissue or liquid containing disrupted tissue, cell culture supernatant, serum, pleural effusion, abdominal dropsy or ocular fluid is reacted with the monoclonal antibody of the present invention and then reacted with a radioactively labeled anti-immunoglobulin antibody or binding fragment thereof, followed by measurement of the radioactivity with a scintillation counter, etc.

Immunological cell staining or immunological tissue staining is a technique in which a sample such as separated cells or liquid containing disrupted cells, separated tissue or liquid containing disrupted tissue, cell culture supernatant, serum, pleural effusion, abdominal dropsy or ocular fluid is reacted with the monoclonal antibody of the present invention and then reacted with an anti-immunoglobulin antibody or a binding fragment thereof labeled with a fluorescent substance such as fluorescein isothiocyanate (FITC) or an enzyme such as peroxidase or biotin, followed by microscopic observation.

### (2) Search for and Identification of Compounds that regulate the Expression of the Polypeptide of the Invention (hereinafter, referred to as "expression-regulating compounds") using the Antibodies of the Invention

Expression-regulating compounds for the polypeptide of the present invention can be searched for and identified by contacting cells expressing the polypeptide of the present invention with a test sample and then using an antibody that recognizes the polypeptide of the present invention. Such compounds present in the cells or a culture supernatant thereof can be searched for and identified.

Cells that may be used for this method include any cell, cell strain or tissue that is expressing the polypeptide of the present invention or the mRNA of the polypeptide; and any cell, cell strain or tissue in which the expression of the polypeptide has been confirmed by an immunological detection method using the antibody described below.

The mRNA of the polypeptide may be detected by conventional methods such as Northern hybridization, dot blot hybridization of RNA, or RT-PCR.

As a probe for hybridization, etc. and as primers for RT-PCT, etc., fragments of the MT4-MMP(2) gene of the present invention may be used when the polypeptide of interest is MT4-MMP(2). Specifically, DNA fragments having a partial sequence selected from the DNA sequence as shown in SEQ ID NO: 3 or SEQ ID NO: 4 may be used preferably. As a preferable example of the above cell strain, a human monocyte strain THP-1 (ATCC TIB-202) may be given.

On the other hand, when the polypeptide of interest is MT5-MMP, fragments of the MT5-MMP gene of the present invention may be used as a probe for hybridization, etc. and as primers for RT-PCT, etc. Specifically, DNA fragments having a partial sequence selected from the DNA sequence as shown in SEQ ID NO: 7 or SEQ ID NO: 8 may be used preferably. As preferable examples of the above cell strain, human fibroblastoma cell line (HT-1080), neuroblastoma cell line (SK-N-SH), undifferentiated-type glioma cell line (no. 10), glioma cell line (KALS-1), pancreatic cancer cell line (PANC-1 or MIA PaCa-2) and hepatoma cell line (SK-HEP-1 or Hep 3B) may be enumerated.

Cells expressing the polypeptide of the present invention are suspended in, for example, a medium in which they can grow. A test sample is added to the medium to thereby contact the sample with the cells. Subsequently, the amount of the polypeptide expressed in the cells is determined based on the method described below using the antibody described in [3] above.

Briefly, cells to be immunologically stained are suspended in a buffer such as immunological cell staining buffer (PBS containing 1% BSA, 0.02% EDTA and 0.05% sodium azide) and added to round-bottom 96-well plates (1-20x10⁵ cells/well).

To the plate, the polyclonal antibody to the polypeptide of the present invention obtained in [3] above, a culture supernatant of the monoclonal antibody-producing hybridoma, the purified monoclonal antibody to the polypeptide of the present invention obtained in [3] above, or the monoclonal antibody labeled with biotin by a known method (Enzyme-Labeled Antibody Technique, Gakusai Kikaku Co., 1985) diluted with the immunological cell staining buffer or this buffer containing 10% animal serum to give a concentration of 0.1-50 *µ* g/mL is added (20-500 *µ* L/well) and left under ice cooling for 30 min.

When the polyclonal antibody to the polypeptide of the present invention obtained in [3], a culture supernatant of the monoclonal antibody-producing hybridoma, or the purified monoclonal antibody to the polypeptide of the present invention obtained in [3] was used in the above operation, the immunological cell staining buffer is added to the plate to wash cells. Then, the immunological cell staining buffer containing an anti-immunoglobulin antibody labeled with a fluorescent substance such as FITC or phycoerythrin at a concentration of about 0.1-50 *µ* g/mL is added to the plate (50-500 *µ* L/well) and left under ice cooling for 30 min while shielding from light. When the monoclonal antibody labeled with biotin was used in the above operation, streptavidin labeled with a fluorescent substance such as FITC or phycoerythrin is added to the plate (50-500 *µ* L/well) and left under ice cooling for 30 min while shielding from light. Subsequently, in both cases, the immunological cell staining buffer is added to the plate to wash cells thoroughly. Then, analysis is carried out with a fluorescent microscope, cell sorter, or the like.

As a test sample, a synthetic compound, naturally occurring protein, artificially synthesized protein, peptide, saccharide, lipid, modified product or derivative of these substances; urine, a body fluid, tissue extract, cell culture supernatant or cell extract from mammals (e.g., mouse, rat, guinea pig, hamster, pig, sheep, bovine, equine, canine, feline, simian, or human); or a non-peptidic compound, fermentation product, or extract from plants or other organisms may be used.

When a peptide is used as a test sample, a random peptide library may be utilized. As a random peptide library, peptides on phage [Proc. Natl. Acad. Sci. USA, 87, 6378 (1990); PCT Patent Application No. 96/40189] or peptides on plasmids (US Patent Nos. 5,270,170 and 5,338,665) may be given.

Expression-regulating compounds may be identified by searching for those test samples that could increase or decrease the content of the polypeptide of the present invention compared to the content in systems to which the test samples were not added.

### (3) Search for and Identification of Inhibitors or Activators of the Polypeptides of the Invention

A test sample is added to a plate coated with the purified polypeptide of the present invention prepared by the method described in [2] above. Then, the antibody of the present invention prepared by the method described in [3] above is added to the plate.

By comparing the effects of test samples upon the binding of the antibody to the polypeptide of the present invention by such methods as ELISA or RIA, substances that bind to the polypeptide of the present invention can be screened from the test samples.

The compounds, which are obtained by this screening, include compounds that inhibit proteinase activity (inhibitors) and compounds that enhance proteinase activity (activators) are included.

As test samples, those substances enumerated in (2) above may be used.

(4) The polypeptide of the present invention present in a cell or tissue of healthy individuals and subjects may be immunologically detected or quantitatively determined using the antibody of the present invention. Then, the amounts of the polypeptide may be compared between healthy individuals and subjects to thereby examine changes in the amount of expression of the polypeptide. Thus, the antibody to MT4-MMP(2) polypeptide may be used to diagnose disease states of subjects such as arthrosis deformans, rheumatoid arthritis, asthma, autoimmune diseases, atopic dermatitis, psoriasis, contact dermatitis, ischemic heart diseases, hepatitis, nephritis, pancreatitis, arteriosclerosis, leukemia, malignant tumor, or inflammations associated with infiltration of leukocytes. The antibody to MT5-MMP polypeptide may be used to diagnose disease states of subjects such as arthrosis deformans, rheumatoid arthritis, asthma, autoimmune diseases, atopic dermatitis, psoriasis, contact dermatitis, ischemic heart diseases, hepatitis, nephritis, pancreatitis, arteriosclerosis, leukemia, malignant tumor, brain disorders at the time of cerebral apoplexy, Alzheimer's disease, dementia, multiple sclerosis, Parkinson's disease, brain tumor, or inflammations associated with infiltration of leukocytes.

(5) By administering an antibody that inhibits the function (proteinase activity) of the polypeptide of the present invention, treatment or prevention of various diseases is expected. When the antibody to MT4-MMP(2) polypeptide is administered, treatment or prevention of diseases such as arthrosis deformans, rheumatoid arthritis, asthma, autoimmune diseases, atopic dermatitis, psoriasis, contact dermatitis, alopecia, ischemic heart diseases, brain disorders at the time of cerebral apoplexy, tissue transplantation, hepatitis, nephritis, pancreatitis, arteriosclerosis, leukemia, malignant tumor, or inflammations associated with infiltration of leukocytes is expected. When the antibody to MT5-MMP polypeptide is administered, treatment or prevention of diseases such as arthrosis deformans, rheumatoid arthritis, asthma, autoimmune diseases, atopic dermatitis, psoriasis, contact dermatitis, alopecia, ischemic heart diseases, brain disorders at the time of cerebral apoplexy, tissue transplantation, hepatitis, nephritis, pancreatitis, arteriosclerosis, leukemia, malignant tumor, Alzheimer's disease, dementia, multiple sclerosis, Parkinson's disease, brain tumor, or inflammations associated with infiltration of leukocytes is expected.

With respect to pharmaceuticals containing the antibodies of the present invention, the antibody may be administered alone as a therapeutic agent. However, usually, it is desirable to use the antibody after mixing it with one or more pharmacologically acceptable carriers and formulating into a pharmaceutical preparation by any of the well known methods in the technical field of pharmacology.

As to the route of administration of the therapeutic agent, it is desirable to use the most effective route for treatment. Oral administration or parenteral administration such as intraoral, intra-respiratory tract, intrarectal, subcutaneous, intramuscular and intravenous administration may be used.

Examples of dosage forms of the therapeutic agent include ointment, spray, capsules, tablets, granules, syrup, emulsion, suppositories, injections, and tapes.

Examples of appropriate preparations for oral administration include emulsion, syrup, capsules, tablets, powder and granules.

Liquid preparations such as emulsion and syrup may be prepared using, as additives, water; saccharides such as sucrose, sorbitol, fructose; glycols such as polyethylene glycol, propylene glycol; oils such as sesame oil, olive oil, soybean oil; preservatives such as p-hydroxybenzoic acid esters; and flavors such as strawberry flavor, pepper mint.

Solid preparations such as capsules, tablets, powder and granules may be prepared using, as additives, excipients such as lactose, glucose, sucrose, mannitol; disintegrating agents such as starch, sodium alginate; lubricating agents such as magnesium stearate, talc; binders such as polyvinyl alcohol, hydroxypropylcellulose, gelatin; surfactants such as fatty acid esters; and plasticizers such as glycerol.

Examples of appropriate preparations for parenteral administration include injections, suppositories and spray.

Injections may be prepared using carriers consisting of, for example, a salt solution, glucose solution or mixture thereof.

Suppositories may be prepared using carriers such as cacao butter, hydrogenated fats or carboxylic acid.

With respect to spray preparations, the antibody of the present invention as it is may be used as a spray. However, it is preferable to formulate the antibody of the present invention into a spray preparation using a carrier that does not stimulate the mucous membranes of the oral cavity and respiratory tract of individuals and that disperses the antibody as fine particles to thereby facilitate absorption.

Examples of such a carrier include lactose and glycerol.

Depending on the natures of the antibody of the present invention and a carrier to be used, preparations such as aerosol or dry powder may be prepared.

In these parenteral preparations, those components enumerated above as additives for oral preparations may also be used.

Dose or the number of times of administration may vary depending on the intended therapeutic effect, the route of administration, the treatment period, the age and body weight of the individual, etc. Usually, 10 *µ* g/kg to 8 mg/kg is administered to an adult per day.

### [5] Use of the Expression-Regulating Compounds

The expression-regulating compounds obtained in (2) in [4] above are expected to be useful for treating or preventing various diseases. Such compounds obtained using the antibodies to MT4-MMP(2) polypeptide are expected to be useful for treating or preventing arthrosis deformans, rheumatoid arthritis, asthma, autoimmune diseases, atopic dermatitis, psoriasis, contact dermatitis, alopecia, ischemic heart diseases, brain disorders at the time of cerebral apoplexy, tissue transplantation, hepatitis, nephritis, pancreatitis, arteriosclerosis, leukemia, malignant tumor, or inflammations associated with infiltration of leukocytes. Such compounds obtained using the antibodies to MT5-MMP polypeptide are expected to be useful for treating or preventing arthrosis deformans, rheumatoid arthritis, asthma, autoimmune diseases, atopic dermatitis, psoriasis, contact dermatitis, alopecia, ischemic heart diseases, brain disorders at the time of cerebral apoplexy, tissue transplantation, hepatitis, nephritis, pancreatitis, arteriosclerosis, leukemia, malignant tumor, Alzheimer's disease, dementia, multiple sclerosis, Parkinson's disease, brain tumor, or inflammations associated with infiltration of leukocytes.

Pharmaceuticals containing the expression-regulating compounds may be formulated into pharmaceutical preparations in the same manner as described in [4] above for the formulation of the pharmaceutical preparations containing the antibodies of the present invention. The resultant pharmaceutical preparations may be administered in the same manner as described in [4] above.

### EXAMPLES

Hereinbelow, the present invention will be described more specifically with reference to the following Examples. However, the scope of the present invention is not limited by these Examples.

### EXAMPLE 1. Cloning of the Gene of Mouse MT4-MMP-Related Protein [MT4-MMP(2)]

Since MT4-MMP gene is highly expressed in the human brain, a brain cDNA library from mouse 17-day embryo was prepared using ZAP-cDNA Synthesis Kit (Stratagene) according to the manual attached to the Kit.

Using a partial sequence of the human MT4-MMP gene (positions 233-1899 of SEQ ID NO: 17) as a probe, the resultant cDNA library was screened by plaque hybridization.

Several of the positive clones that hybridized to the above probe were analyzed for their nucleotide sequences. All of the analyzed clones contained a signal peptide sequence that is considered missing in the reported human MT4-MMP gene; the longest clone was 3.5 kb. Therefore, it was considered that an mRNA corresponding to the DNA of SEQ ID NO: 3 which can express the 587 amino acid MT4-MMP(2) shown in SEQ ID NO: I is expressed in mouse.

### EXAMPLE 2. Cloning of Human MT4-MMP (2) Gene

EST clones relating to the human MT4-MMP gene were searched for through databases. However, no clones were registered which contain a sequence encoding a signal peptide as seen in the above-mentioned mouse gene. Therefore, it was considered that secretion-type human MT4-MMP gene does not exist or there are reasons that make the isolation thereof difficult.

A human brain cDNA library (Clontech) was screened using a partial sequence of the mouse MT4-MMP(2) gene, as a probe, from Example 1 encoding an N-terminal region representing the signal peptide. However, the gene of interest could not be isolated. Then, the inventors analyzed 5' regions of transcripts by 5' RACE. For this analysis, monocyte-derived THP-1 (ATCC TIB-202; American Type Culture Collection) cells were used in which expression of MT4-MMP mRNA had been confirmed.

Briefly, a cDNA was prepared using poly(A)⁺ RNA isolated from human THP-1 cells, a human MT4-MMP selective primer (SEQ ID NO: 9) and Superscript II (Gibco BRL). A single-stranded oligonucleotide adaptor (SEQ ID NO: 10) was ligated to the resultant cDNA with T4 RNA ligase. Then, a PCR was performed in GC buffer using the MT4-MMP selective primer (SEQ ID NO: 9), an adaptor selective primer (SEQ ID NO: 11) and LA Taq (Takara). After completion of this reaction, another PCR was performed using a gene-selective, other primer (SEQ ID NO: 12) and an adaptor selective primer (SEQ ID NO: 13).

The analysis of the 50 clones revealed that, while 3 clones were cDNA fragments containing an MT4-MMP sequence, 47 clones were cDNA fragments encoding a signal peptide sequence similar to that in mouse MT4-MMP(2). From this, in addition to the downstream region of the propeptide sequence already known, the entire region of the mRNA of SEQ ID NO: 4 encoding human MT4-MMP(2) as shown in SEQ ID NO: 2 containing a signal peptide has been elucidated. Although the nucleotide sequence of an EST clone H97792 was almost identical with the sequence of the MT4-MMP gene reported by Puente [Cancer Research, 56, 944 (1996)], a partial sequence of the catalytic domain was different. The EST clone H97792 was more highly conserved with mouse MT4-MMP(2) gene. When the entire sequence of human MT4-MMP(2) gene was determined newly, differences were found even in the previously sequenced region of the MT4-MMP gene reported by Puente [Cancer Research, 56, 944 (1996)].

Mouse and human MT4-MMP(2) genes are mutually conserved well; their propeptide domains, catalytic domains, hinge domains and hemopexin-like domains had 87%, 87%, 78% and 96% homology, respectively. Their signal peptide domains and transmembrane domains had relatively low similarities of 54% and 35%, respectively. When the catalytic domain of human MT4-MMP(2) gene were compared with the catalytic domains of MT1-MMP, MT2-MMP and MT3-MMP, the similarities were 36%, 39% and 31%, respectively. These results also supported that mouse MT4-MMP(2) gene is most close to human MT4-MMP(2) gene. Thus, it was concluded that mouse MT4-MMP(2) gene is a mouse homologue to human MT4-MMP(2) gene.

### EXAMPLE 3. Expression of MT4-MMP(2) and Detection of the Gene Product

In order to confirm that a gene product is certainly translated from the isolated cDNA, the cDNA was integrated into pSG5 vector (Stratagene) containing an SV40 promoter. For detecting the expressed product, a FLAG sequence (Eastman Chemical) was integrated downstream of the latent enzyme processing site to thereby enable detection with anti-FLAG antibodies.

COS-1 cells were transfected with mouse or human MT4-MMP(2) expression plasmids. After 48 hr, cells were harvested and lysed followed by detection of FLAG-labeled MT4-MMP by Western blotting. With the use of an anti-FLAG antibody M2 (Eastman Chemical), a specific 66 kDa band in both cells transfected with the expression plasmids was detected.

### EXAMPLE 4. Detection and Analysis of MT4-MMP Transcript

Since MT4-MMP transcript has an *Alu* sequence at 5' end, there was a possibility that it contains intron(s). Using a partial sequence of human MT4-MMP(2) (positions 212-519 of SEQ ID NO: 4) as a probe, hybridized clones were isolated from a library of Health Science Research Resources Bank (Deposit No. LI020) by hybridization, and plasmids were extracted from the resultant clones by conventional methods. Then, the present inventors examined nucleotide sequences around the 5' end region (positions 140-272 of SEQ ID NO: 17) of MT4-MMP contained in these plasmids.

When MT4-MMP gene was compared with MT4-MMP(2) gene, MT4-MMP nucleotide sequence of the region in which homology no longer exists (positions 1-139 of SEQ ID NO: 17) was almost identical with positions 3008-3147 of the genomic sequence (SEQ ID NO: 18); and a splice donor sequence was found on the border between the region with homology and the region without homology. The sequence encoding the exons of MT4-MMP (positions 140-340 of SEQ ID NO: 17) were almost identical with positions 3148-3280 and positions 3564-3633 of the genomic sequence (SEQ ID NO: 18). From these results, it was concluded that the transcript still containing the first intron is MT4-MMP transcript.

From these results, it was considered that two mRNAs encoding MT4-MMP and MT4-MMP(2) are expressed in human.

In order to discriminate these two transcripts by performing RT-PCR separately, 5' primers specific to individual transcripts (MT4-MMP: SEQ ID NO: 14; MT4-MMP(2): SEQ ID NO: 15) and a common 3' primer (SEQ ID NO: 16) were prepared.

The expression of these transcripts in various cancer cells is shown in Table 1 below.

**Table 1.**

| Expression of MT4-MMP(2) and MT4-MMP Transcripts in Cancer Cells | | | |
|---|---|---|---|
| Cancer Cell Line | MT4-MMP(2) | MT4-MMP | Accession Number |
| Jurkat (T cell) | ++ | +/- | ATCC TIB-152 |
| Raji (B cell) | - | - | ATCC CCL-86 |
| BJAB (B cell) | - | - | ATCC HB-136 |
| THP-1 (monocytic) | ++ | + | ATCC TIB-202 |
| K562 (monocytic) | ++ | - | ATCC CCL-243 |
| U-937 (monocytic) | ++ | - | ATCC CRL-1593.2 |
| U-251 MG (astrocytoma) | ++ | - | Hakkoken IFO50288 |
| SK-N-SH (neuroblastoma) | ++ | - | ATCC HTB-11 |
| no.10 (glioma) | +/- | - | Hakkoken IFO50368 |
| KALS-1 (glioma) | ++ | - | Hakkoken IFO50434 |
| MKN-7 (gastric) | + | - | Riken RCB0999 |
| MKN-28 (gastric) | - | - | Riken RCB1000 |
| NUGC-4 (gastric) | + | - | HS Found JCRB0834 |
| PANC-1 (pancreatic) | ++ | + | ATCC CRL-1469 |
| MIA PaCa-2 (pancreatic) | ++ | +/- | ATCC CRL-1420 |
| SK-HEP-1 (hepatoma) | ++ | + | ATCCHTB-52 |
| Hep 3B (hepatoma) | ++ | + | ATCC HB-8064 |
| ZR-75-1 (breast) | ++ | + | ATCC CRL-1500 |
| MCF7(adenocarcinoma) | ++ | + | ATCC HTB-22 |
| T-24 (bladder) | ++ | + | ATCC HTB-4 |
| A375 (melanoma) | ++ | + | ATCC CRL-1619 |
| HT-1080 (fibrosarcoma) | + | - | ATCC CCL-121 |
| ++: strong expression; +: medium expression; +/-: slight expression; -: no expression ATCC: American Type Culture Collection HS Found.: Japan Health Sciences Foundation Riken: The Institute of Physical and Chemical Research Hakkoken: Institute for Fermentation, Osaka | | | |

MT4-MMP was only expressed in those cells where expression of MT4-MMP(2) was recognized.

From these results, it is believed that MT4-MMP(2) is the major transcript and that expression of MT4-MMP also occurs depending on cells under similar transcriptional control.

### EXAMPLE 5. Expression of MT4-MMP(2) in Mouse Tissues

Tissues of 4-week old mice were excised by organ. RNA was extracted therefrom and used to examine the expression pattern of MT4-MMP(2). Briefly, 20 *µ* g of total RNA was electrophoresed on 1% agarose gel and transferred onto a nylon membrane followed by Northern blot analysis using ³²P-labeled mouse MT4-MMP(2) gene as a probe, to thereby examine the expression pattern of MT4-MMP(2).

Organs in which particularly high expression was observed were the cerebrum, cerebellum, brainstem, large intestine, uterus, and testis. Little expression was observed in the kidney, mammary gland, and placenta. The results of expression in mouse were consistent with the results of MT4-MMP expression in human tissues reported by Puente et al. [Cancer Research, 56, 944 (1996)].

In mouse, the expression of MT4-MMP(2) was very high in the brain, and its expression was also observed in some limited organs such as the large intestine, uterus and testis. This presents a contrast to the expression of MT1-MMP and MT2-MMP seen in a relatively wide range of tissues. From this, it is believed that MT4-MMP(2) is involved in the maintenance of homeostasis in tissues through the degradation of extracellular substrates specific to those organs expressing MT4-MMP(2).

### EXAMPLE 6. Expression of a Mouse MT4-MMP(2) Partial Peptide (Hemopexin-like Domain) in E. coli

A cDNA encoding a mouse MT4-MMP(2) partial peptide (hemopexin-like domain) having an amino acid sequence represented by positions 321-550 of SEQ ID NO: 1 to which a methionine residue was added at the N-terminus was amplified by polymerase chain reaction (PCR) using the cDNA of mouse MT4-MMP(2) as a template.

The amplified fragment was subcloned into an *E*. *coli* expression vector pET3a (Takara) and then introduced into *E*. *coli* BL21 (DE3) pLysS (Takara). This *E*. *coli* was grown in 1 liter of expression medium in the presence of 100 *µ* g/mL of ampicillin until OD₆₀₀ reached 0.5. Then, the cells were stimulated with 0.4 mmol/L of isopropyl- β -D-thiogalactopyranoside (IPTG) and cultured for another three hours.

After the culturing, insoluble bodies (inclusion bodies) consisting of the mouse MT4-MMP(2) partial peptide formed in E. *coli* cells were collected by conventional methods and dissolved in a solubilization solution containing 8 mol/L urea, 50 mmol/L Tris-HCl (pH 8.6) and 20 mmol/L dithiothreitol (DTT). The resultant solution was applied to High Q anion exchange column followed by recovery of the fraction eluted with 0.2 mol/L sodium chloride.

This fraction was diluted with a solution containing 50 mmol/L Tris-HCl (pH 8.6), 6 mol/L urea, 1 mmol/L dithiothreitol, 0.15 mol/L sodium chloride, 5 mmol/L calcium chloride, 100 mmol/L zinc chloride and 0.02% sodium azide. Then, cystamine (final concentration: 20 mmol/L) was added to the resultant dilution. Subsequently, the resultant solution was dialyzed against a solution containing 50 mmol/L Tris-HCl (pH 8.6), 0.15 mol/L sodium chloride, 5 mmol/L calcium chloride, 100 mmol/L zinc chloride, 5 mmol/L β mercaptoethanol, 1 mmol/L 2-hydroxyethyldisulfide and 0.02% sodium azide at 4°C. Further, dialysis was performed against 10 volumes of a solution containing 50 mmol/L Tris-HCl (pH 7.5), 0.15 mol/L sodium chloride, 5 mmol/L calcium chloride, 50 mmol/L zinc chloride and 0.02% sodium azide (4 hr x 3 times). The dialyzed solution was centrifuged at 22,000xg at 4°C for 10 min to remove the precipitate.

The supernatant was applied to S-200 column pre-equilibrated with a buffer containing 50 mmol/L Tris-HCl (pH 7.5), 150 mmol/L sodium chloride, 10 mmol/L calcium chloride and 0.02% sodium azide for gel filtration to obtain a mouse MT4-MMP(2) partial peptide corresponding to the hemopexin-like domain. The thus obtained partial peptide was used as an antigen for preparing antibodies.

### EXAMPLE 7. Preparation of Polyclonal Antibodies that Recognize Mouse MT4-MMP(2)

The mouse MT4-MMP(2) partial peptide (100 *µ* g) prepared in Example 6 was administered to two rabbits (Japanese White Rabbits) together with complete Freund's adjuvant. From two weeks after the initial administration, 100 *µ* g of the mouse MT4-MMP(2) partial peptide was administered once a week together with incomplete Freund's adjuvant 6 times in the total.

Blood samples were taken from the ear microvein. Then, serum antibody titers of the samples were examined by the enzyme immunoassay as described in (2) in Example 8 below. Then, serum was collected from rabbits that exhibited a sufficient antibody titer by exsanguination. The resultant serum was purified to IgG fraction by the method as described in (5) in Example 8 below. This fraction was used as a polyclonal antibody.

### EXAMPLE 8. Preparation of Monoclonal Antibodies that Recognize Mouse MT4-MMP(2)

### (1) Immunization of Animals and Preparation of Antibody-Producing Cells

The mouse MT4-MMP(2) partial peptide (50 *µ* g) prepared in Example 6 was administered to 5-week old female SD rats together with 2 mg of aluminium hydroxide adjuvant (Antibodies - A Laboratory Manual, Cold Spring Harbor Laboratory, p. 99, 1988) and 1x10⁹ cells of pertussis vaccine (Serum Institute, Chiba Pref., Japan).

From two weeks after the initial administration, 50 *µ* g of the mouse MT4-MMP(2) partial peptide was administered once a week 4 times in the total. Blood samples were taken from the venous plexus of the eyeground, and serum antibody titers of the samples were examined by the enzyme immunoassay as described below. Three days after the final immunization, the spleen was removed from rats that exhibited a sufficient antibody titer.

The spleen was cut into pieces in MEM (Minimum Essential Medium) (Nissui Pharmaceutical), loosened with tweezers and centrifuged (at 250 xg for 5 min). To the resultant precipitate fraction, Tris-ammonium chloride buffer (pH 7.6) was added to treat it for 1 to 2 min. to thereby remove erythrocytes. The resultant precipitate fraction (cell fraction) was washed with MEM medium 3 times and then used in cell fusion.

### (2) Enzyme Immunoassay

As an antigen for this assay, the mouse MT4-MMP(2) partial peptide obtained in Example 6 was used. As control antigens, *E*. *coli-*expressed human MT1-MMP hemopexin-like domain (hereinafter, abbreviated to "human MT1-MMP") prepared in the same manner as described in Example 6 and a protein from *E*. *coli* cells were used.

Each of the antigens was dispensed at a concentration of 10 *µ* g/mL and added to all wells of 96-well ELISA plates (50 *µ* L/well), and left overnight at 4°C for adsorption. After the plate was washed, 1% bovine serum albumin (BSA)/Dulbecco's phosphate buffer (phosphate buffered saline: PBS) was added thereto (100 *µ* L/well) and left at room temperature for one hour to thereby block the remaining active groups.

Subsequently, the 1% BSA/PBS was discarded from the plate. An immunized rat anti-serum, a culture supernatant of anti-mouse MT4-MMP(2) monoclonal antibody, or a purified monoclonal antibody was added to the plate (100 *µ* L/well) and left for two hours. After the plate was washed with 0.05% polyoxyethylene (20) sorbitan monolaurate [equivalent to Tween™ 20 of ICI; manufactured by Wako Purechemical Industries]/PBS (hereinafter, referred to as Tween-PBS), a 200-fold dilution of peroxidase-labeled rabbit anti-rat immunoglobulin (DAKO) was added (50 *µ* L/well) and then left at room temperature for one hour. After the plate was washed with Tween-PBS, an ABTS [ammonium 2,2-azino-bis(3-ethylbenzothiazol-6-sulfonate)] substrate solution [1 mmol/L ABTS/0.1 mol/L citrate buffer (pH 4.2)] was added for color development. The absorbance at 415 nm (OD₄₁₅) was measured with a plate reader (Emax; Molecular Devices).

### (3) Preparation of Mouse Myeloma Cells

An 8-azaguanine resistant mouse myeloma cell line P3X63Ag8U.1 (P3-U1; purchased from ATCC) was cultured in a normal medium to secure 2x10⁷ cells or more at the time of cell fusion. This cell line was supplied to cell fusion as a parent cell line.

### (4) Preparation of Hybridomas

The rat spleen cells obtained in (1) above and the myeloma cells obtained in (3) above were mixed to give a ratio of 10:1 and centrifuged (at 250 xg for 5 min). Cell pellet in the resultant precipitate fraction was loosened thoroughly. Then, a liquid mixture composed of 2 g of polyethylene glycol-1000 (PEG-1000), 2 mL of MEM medium and 0.7 mL of dimethylsulfoxide was added to the cells in an amount of 0.2-1 mL per 10⁸ rat spleen cells at 37°C while stirring. To the suspension, 1-2 mL of MEM medium was added every one to two minutes (several times in the total). Then, MEM medium was added thereto to make the total volume 50 mL.

This suspension was centrifuged (at 900 rpm for 5 min) to obtain a precipitate fraction. Cells in this fraction were loosened and then gently suspended in 100 mL of HAT medium [10% fetal bovine serum-supplemented RPMI medium to which HAT Media Supplement (Boehringer Mannheim) was added] by pipetting gently with a measuring pipette. The resultant suspension was added to 96-well culture plates (200 *µ* L/well) and cultured in a 5% CO₂ incubator at 37°C for 10-14 days.

After the culturing, the culture supernatant was examined by the enzyme immunoassay described in (2) above to thereby select those wells that reacted with the mouse MT4-MMP(2) partial polypeptide but did not react with human MT1-MMP nor the *E*. *coli*-derived protein. Cells contained in the selected wells were subjected to cloning twice by limiting dilution technique. As a result, anti-mouse MT4-MMP(2) monoclonal antibody-producing hybridomas KM2560, KM2561, KM2562, KM2563, KM2564 and KM2565 were obtained. (Of these, KM2560, KM2561 and KM2562 were deposited as FERM BP-6730, FERM BP-6731 and FERM BP-6732, respectively, at the National Institute of Bioscience and Human-Technology, Agency of Industrial Science and Technology on May 27, 1999.) (Fig. 1)

### (5) Purification of Monoclonal Antibodies

Each of the hybridoma clones obtained in (4) above was administered to pristane-treated 8-week old nude female mice (BALB/c) by intraperitoneal injection (5-20 x 10⁶ cells/mouse). Ten to 21 days after the administration, abdominal dropsy was collected (1-8 mL/mouse) from those mice in which abdominal dropsy had been accumulated as a result of the conversion of the hybridoma into abdominal dropsy cancer.

The abdominal dropsy was centrifuged (at 1200 xg for 5 min) to remove solid matter. Purified IgM monoclonal antibodies were obtained by salting out the resultant abdominal dropsy with 50% ammonium sulfate, dialyzing against PBS to which 0.5 mol/L sodium chloride had been added, and then applying to a column packed with Cellulofine GSL2000 (Seikagaku Corp.) (bed volume: 750 mL) at a flow rate of 15 mL/hr to thereby collect the IgM fraction.

Purified IgG monoclonal antibodies were obtained by purification using the caprylic acid precipitation technique [Antibodies - A Laboratory Manual, Cold Spring Harbor Laboratory, 1988]. The subclasses of the antibodies were determined by ELISA using a subclass typing kit. The results are shown in Table 2.

**Table 2.**

| Antibody Classes of Anti-Mouse MT4-MMP(2) Monoclonal Antibody-Producing Hybridomas KM2560-2565 | |
|---|---|
| KM No. | Antibody Class |
| KM2560 | IgM |
| KM2561 | IgG2a |
| KM2562 | IgG2a |
| KM2563 | IgM |
| KM2564 | IgM |
| KM2565 | IgM |

### (6) Western Blotting

The reaction specificities of the anti-mouse MT4-MMP(2) monoclonal antibodies selected in (4) above were examined by Western blotting.

The mouse MT4-MMP(2) partial peptide obtained in Example 6 (0.1 *µ* g/lane), human MT1-MMP (0.4 *µ* g/lane), or the *E*. coli-derived protein (0.1 *µ* g/lane) was fractionated by SDS-polyacrylamide electrophoresis (SDS-PAGE: 5-20% gradient gel; Atto) [Antibodies - A Laboratory Manual, Cold Spring Harbor Laboratory, 1988] and blotted on a PVDF membrane (Millipore).

After the membrane was blocked with 1% BSA-PBS, a culture supernatant of each of the anti-mouse MT4-MMP(2) monoclonal antibodies and the rabbit polyclonal antibody obtained in Example 7 were added to the membrane and left at room temperature for 2 hr. After the membrane was washed with Tween-PBS thoroughly, a 1000-fold dilution of peroxidase-labeled rabbit anti-rat immunoglobulin antibody (DAKO) was added to the membrane as the secondary antibody and left at room temperature for 1 hr. When the polyclonal antibody was used, it was used in the same manner as the monoclonal antibody after dilution to 20 *µ* g/mL with 1% BSA/PBS. As the secondary antibody, a 1000-fold dilution of peroxidase-labeled pig anti-rabbit immunoglobulin antibody (DAKO) was used.

After the membrane was washed with Tween-PBS thoroughly, reaction was detected using ECL kit (Amersham Pharmacia Biotech) to thereby confirm that the anti-mouse MT4-MMP(2) monoclonal antibodies KM2560-2565 and the rabbit polyclonal antibody react specifically with a 26 kDa band corresponding to the molecular weight of the mouse MT4-MMP(2) partial peptide (Fig. 2).

### EXAMPLE 9. Transfer of Human MT4-MMP(2) Gene into COS-1 Cell Line

As a host cell for gene transfer, simian kidney-derived COS-1 cells (ATCC CRL-1650) purchased from ATCC were used. Ten milliliters of suspension of cultured COS-1 cells adjusted to give a concentration of 2x10⁵ cells/mL were added to a 10 cm culture dish and cultured overnight. Then, using FuGENE™6 transfection reagent (Boehringer Mannheim), gene transfer was carried out as described below.

First, a full-length cDNA of human MT4-MMP(2) gene was subcloned into an expression vector pSG5 (Stratagene) and then introduced into *E. coli* XL-1 Blue MRF'. This *E*. *coli* was cultured in 150 mL of LB (Luria-Bertani) medium in the presence of 100 *µ* g/mL ampicillin followed by purification of the plasmid DNA using the AX500 cartridge of NucleoBond Plasmid Kit (Clontech).

A serum-free medium OPTI-MEM^{R}I (Lifetech Oriental) (816 *µ* L) was placed in a plastic tube, to which 24 *µ* L of FuGENE was added and left stationary at room temperature for 5 min. To this solution, 12 *µ* L of a solution of the above-described plasmid DNA from human MT4-MMP(2) diluted to 1 *µ* g/ *µ* L with Tris-EDTA (pH 8.0) was added, mixed gently and left stationary for 15 min. This plasmid solution (852 *µ* L) was added to cultured COS-1 cells that had been cultured since the previous day. After the culture was made homogeneous, the cells were cultured for 3 days.

### EXAMPLE 10. Detection of Human MT4-MMP(2) Expression in COS-1 Cells by Immunological Staining

The culture supernatant of the thus gene-transferred COS-1 cells was removed, and PBS was added to the cells. Then, the cells were collected from the culture plate with a cell scraper (Sumitomo Bakelite) and suspended in PBS to give a concentration of 2x10⁵ cells/mL. Five hundred *µ* L of this cell suspension was adhered to a silane-coated slide glass (Matsunami) by centrifuging at 40 xg for 3 min with Site Spin 3 (SHANDON).

The cells were fixed with 4% paraformaldehyde (Wako Purechemical Industries) at 4°C for 15 min and then washed 3 times with PBS for 3 min each time (hereinafter, this washing process is abbreviated to "washing"). In order to eliminate non-specific reactions, 100 *µ* L of a blocking reagent (DAKO) was added thereto and incubated at room temperature for 1 hr. After removal of the blocking reagent, 100 *µ* L of a culture supernatant of the hybridoma against mouse MT4-MMP(2) (KM2561 or KM2562) or a culture supernatant of a control antibody-producing hybridoma (KM1764 or rat IgG2a) was added thereto as a primary antibody and incubated overnight at room temperature. Then, washing was carried out. Subsequently, 100 *µ* L of biotin-labeled anti-rat immunoglobulin antibody (DAKO; containing 0.25% normal rabbit serum) diluted to 6 *µ* g/mL was added to the cells and reacted at room temperature for one hour.

After washing, in order to inactivate the endogenous peroxidase, the cells were incubated with 0.3% aqueous solution of hydrogen peroxide and 0.1 mol/L sodium azide at room temperature for 30 min. After washing, 100 *µ* L of avidin-biotin complex (manufactured by VECTOR) prepared 30 min before the use was added and reacted at room temperature for 30 min. After washing, the expression of mouse MT4-MMP(2) was detected using diaminobenzidine (manufactured by VECTOR). As counter-staining, hematoxylin staining was performed and the results were observed. As a negative control, untreated COS-1 cells to which the MT4-MMP(2) plasmid DNA had not been added were used. As shown in Fig. 3, specific reactions with KM2561 and KM2562 were recognized in human MT4-MMP(2) gene-transferred COS-1 cells.

### EXAMPLE 11. Detection of Human MT4-MMP(2) Polypeptide by Fluorescein Antibody Technique

The culture supernatants of the above-described gene-transferred COS-1 cells and untreated COS-1 cells were removed, respectively, and PBS was added to both cells. Then, the cells were collected from the culture plate with a cell scraper (Sumitomo Bakelite) and suspended in PBS to give a concentration of 1x10⁶ cells/mL. One microliter of this cell suspension was placed in a plastic tube and centrifuged. Then, the supernatant was removed by suction. For the fixation of the cells, FIX & PERM Cell Permeabilization Kits (Caltag) were used. To the fixed cells, 50 *µ* L of anti-MT4-MMP(2) antibody (culture supernatant of KM2561 or KM2562) was added, agitated and reacted at 4°C for 1 hr.

After washing by centrifugation with PBS, 50 *µ* L of 100-fold dilution of biotin-labeled rabbit anti-rat immunoglobulin antibody (DAKO) was added and reacted at 4°C for 1 hr. After washing by centrifugation with PBS, 50 *µ* L of 200-fold dilution of streptavidin-FITC (Streptavidin-Fluorescein isothiocyanate conjugate; Pharmingen) was added and reacted at 4°C for 30 min. After washing by centrifugation with PBS, fluorescence was measured with FACScan (Becton Dickinson). As shown in Fig. 4, human MT4-MMP(2) polypeptide expressed in the gene-transferred COS-1 cells was detected by KM2561 and KM2562. The axis of ordinates represents cell count, and the axis of abscissas (FL1-H) represents-fluorescence intensity. The dotted line represents the pattern when control [KM1764 which does not recognize MT4-MMP(2)] was added instead of MT4-MMP(2)-recognizing monoclonal antibody, and the solid line represents the pattern when anti-mouse MT4-MMP(2) monoclonal antibody KM2561 or KM2562 was added.

### EXAMPLE 12. Detection of Human MT4-MMP(2) Polypeptide by Western Blotting

The cell lines described below were centrifuged to collect cells. After removal of the supernatant, a solubilization solution [50 mmol/L HEPES, 250 mmol/L sodium chloride, 1 % NP40, 1 mmol/L DTT, 1 mmol/L phenylmethylsulfonyl fluoride, 5 *µ* g/mL leupeptin] was added to the cell pellet and mixed well. The mixture was left stationary at 4°C for 10 min. Then, the mixture was centrifuged at 7000 xg for 30 min to recover the supernatant. This supernatant was used as a sample.

After the amount of protein in the sample was quantitatively determined using a protein determination reagent [Protein Assay; Bio-Rad], the sample (100 *µ* g/lane) was separated by SDS-PAGE (7.5% acrylamide). Then, Western blotting was carried out using anti-mouse MT4-MMP(2) monoclonal antibody KM2561 in the same manner as described in (6) in Example 8. The cell lines used in this experiment were U957 (human histiocytic lymphoma), THP-1 (human monocyte) and Jurkat (human acute T cell leukemia), all of which were purchased from ATCC. As shown in Fig. 5, human MT4-MMP(2) polypeptide was detected around the 68 kilodalton (kd) marker in any of these cell lines.

### EXAMPLE 13. Cloning of Mouse MT5-MMP Gene

In order to isolate mouse MT3-MMP gene, a brain cDNA library from mouse 17-day embryo was prepared using ZAP-cDNA Synthesis Kit (Stratagene) according to the manual attached to the kit.

The resultant cDNA library was screened by plaque hybridization using human MT3-MMP gene as a probe. Clones exhibiting a strong signal and clones exhibiting a weak signal were obtained. The nucleotide sequences of these clones were determined.

As a result of analysis of clones with a weak signal, a 2.1 kb sequence was found in one of them. Although this sequence exhibited weak homology to human and rat MT3-MMP genes, it was not homologous to other MMP genes. Thus, it was considered that this sequence represents a novel MMP gene.

Subsequently, a 3.7 kb cDNA fragment that hybridized to the above-described 2.1 kb sequence was obtained from the above library by plaque hybridization. From the 2.1 kb and 3.7 kb sequences, a 4.2 kb cDNA sequence shown in SEQ ID NO: 7 was obtained.

A protein with 618 amino acids represented by SEQ ID NO: 5 was encoded in the cDNA shown in SEQ ID NO: 7. Since the peptide of SEQ ID NO: 5 contains those sequences corresponding to the individual domains of MT-MMPs in well-conserved states, it was concluded that this peptide is a novel MT-MMP, namely, mouse MT5-MMP (Fig. 6).

### EXAMPLE 14. Cloning of Human MT5-MMP Gene

In order to confirm the human gene corresponding to mouse MT5-MMP gene, a human kidney cDNA library (Clontech) was screened by plaque hybridization using mouse MT5-MMP gene as a probe in the same manner as in Example 9. As a result, a gene that has 92% homology to mouse MT5-MMP gene and is different from known MT-MMP genes was obtained.

All of the sequenced human MT5-MMP cDNA clones lacked a 5' region that is supposed to encode a signal peptide. Thus, the sequence of the missing region was determined by 5' RACE as described below to thereby determine the nucleotide sequence containing the entire region encoding human MT5-MMP gene.

Briefly, cDNA was prepared from a human brain poly(A)⁺ RNA (Clontech) using Superscript II (Gibco BRL) and a human MT5-MMP gene-selective primer (SEQ ID NO: 19) according to the manual attached to the kit.

A single-stranded oligonucleotide adaptor (SEQ ID NO: 10) was ligated to the resultant cDNA with T4 RNA ligase. Then, the cDNA was subjected to PCR in GC buffer using the MT5-MMP gene-selective primer (SEQ ID NO: 19), an adaptor-selective primer (SEQ ID NO: 11) and LA Taq (Takara).

After completion of the above PCR, another PCR was performed using an other gene-selective primer (SEQ ID NO: 20) and an adaptor-selective primer (SEQ ID NO: 13). From the above-mentioned sequence obtained from the human kidney cDNA library using the mouse gene as a probe and the sequence obtained from the 5' RACE, a 2.6 kb cDNA fragment (shown in SEQ ID NO: 8) that encodes a 645 amino acid protein (shown in SEQ ID NO: 6) was obtained.

### EXAMPLE 15. Expression of MT5-MMP mRNAs in Internal Organs

Expression of MT5-MMP gene in tissues was examined by Northern blotting.

Briefly, 20 *µ* g of total RNA was electrophoresed on 1 % agarose gel and transferred onto a nylon membrane. Then, Northern blotting was carried out using ³²P-labeled mouse MT5-MMP gene as a probe to examine the expression pattern of approximately 4 kb MT5-MMP mRNAs.

In 2-week old mice, a strong expression was observed only in the brain; but the expression was around detection limit or below in tissues of other organs.

When expression in human tissues was examined with Multiple Tissue Blot (Clontech) using human MT5-MMP gene as a probe, high expression was observed in the brain. The results of Northern blotting using ³²P-labeled human MT5-MMP gene as a probe revealed that strong expression of both 4.0 kb and 4.8 kb MT5-MMP mRNAs are also recognized in the human brain. In human, the expression was also recognized in the kidney and pancreas. The 4.8 kb mRNA and the 4.0 kb mRNA were expressed strongly in the brain and in the kidney and pancreas, respectively.

Then, RT-PCR was carried out using MT5-MMP specific primers (SEQ ID NOS: 21 and 22) to analyze these fragments. As a result, it was found that a DNA fragment of the same size as that of the fragment amplified in the brain is amplified in the kidney and pancreas with almost equal efficiencies and that no products of different sizes were found. Thus, it was believed that the shorter transcript contains the entire coding region.

When the expression of MT5-MMP in mouse and human was examined, characteristic expression was observed in the brain. In particular, the expression was limited in the brain in mouse, and was very low in other internal organs.

Since the expression of this gene in the brain is characteristic, site-specific expression was examined using Human Brain Multiple Tissue Blot (Clontech).

High expression of MT5-MMP was observed in the cerebellum. Its expression was also observed in the cerebral cortex, medulla, occipital region of head, frontal region of head, temporal region of head and putamen, but not observed in the spinal cord.

These results show a remarkable characteristic of MT5-MMP gene different from other MT-MMP genes expressed in various tissues.

In human, the expression of MT5-MMP was strong in the brain, and its expression was also observed in the kidney and pancreas. The results of examination of its site-specific expression in the human brain revealed a characteristic expression in the cerebellum. High expression in the cerebellum was also confirmed in mouse.

These results suggest the possibility that MT5-MMP controls the degradation of extracellular matrixes around cells associated with such processes as the maturation and maintenance of brain tissues, the construction of nervous network, and so forth.

### EXAMPLE 16. Expression of MT5-MMP mRNA in Cancer Cells

MT1-MMP is expressed frequently in cancer cells *per se* and interstitial cells around them in many cancer tissues and functions as an activator of gelatinase A at the tissue level. The expression of MT5-MMP in various cancer cell strains was examined by RT-PCR using MT5-MMP-specific primers (SEQ ID NOS: 21 and 22).

The results are shown in Table 3 below.

**Table 3.**

| Expression of MT5-MMP Transcript in Cancer Cells | | |
|---|---|---|
| Cancer Cell Line | MT5-MMP | Accession Number |
| Jurkat (T cell) | - | ATCC TIB-152 |
| Raji (B cell) | - | ATCC CCL-86 |
| BJAB (B cell) | - | ATCC HB-136 |
| THP-1 (monocytic) | - | ATCC TIB-202 |
| K562 (monocytic) | - | ATCC CCL-243 |
| U-937 (monocytic) | - | ATCC CRL-1593.2 |
| U-251 MG (astrocytoma) | - | Hakkoken IFO50288 |
| SK-N-SH (neuroblastoma) | +++ | ATCC HTB-11 |
| no.10 (glioma) | ++ | Hakkoken IFO50368 |
| KALS-1 (glioma) | +++ | Hakkoken IFO50434 |
| MKN-7 (gastric) | + | Riken RCB0999 |
| MKN-28 (gastric) | - | Riken RCB1000 |
| NUGC-4 (gastric) | + | HS Found JCRB0834 |
| PANC-1 (pancreatic) | + | ATCC CRL-1469 |
| MIA PaCa-2 (pancreatic) | + | ATCC CRL-1420 |
| SK-HEP-1 (hepatoma) | + | ATCC HTB-52 |
| Hep 3B (hepatoma) | + | ATCC HB-8064 |
| ZR-75-1 (breast) | ? | ATCC CRL-1500 |
| MCF7(adenocarcinoma) | - | ATCC HTB-22 |
| T-24 (bladder) | - | ATCC HTB-4 |
| A375 (melanoma) | +/- | ATCC CRL-1619 |
| HT-1080 (fibrosarcoma) | +/- | ATCC CCL-121 |
| +++: very strong expression; ++: strong expression; +: medium expression; +/-: slight expression; -: no expression ATCC: American Type Culture Collection HS Found.: Japan Health Sciences Foundation Riken: The Institute of Physical and Chemical Research Hakkoken: Institute for Fermentation, Osaka | | |

While MT1-MMP is expressed in various cancer cell lines, cell lines expressing MT5-MMP were specific in the nervous system-derived neuroblastoma [SK-N-SH (HTB-11, ATCC)], undifferentiated-type glioma [no. 10 (IFO50368, Institute for Fermentation, Osaka)], and glioma [KALS-1, (IFO50434, Institute for Fermentation, Osaka)], with the correlation of the high expression in brain.

Also, its expression in pancreatic cancer cell lines [PANC-1 (CRL-1469, ATCC); MIA PaCa-2 (CRL-1420, ATCC)] and hepatoma cell lines [SK-HEP-1 (HTB-52, ATCC): Hep 3B (HB-8064, ATCC)] was characteristic.

It is considered that abnormal expression of MT-MMPs on cell surfaces promotes the infiltration of cells. Actually, excessive expression of MT1-MMP enhances the infiltrating ability of cancer cell lines and increases the frequency of experimental metastasis. In human cancer tissues, cancer cells and fibroblasts around them express MT1-MMP at high frequency, and the presence of gelatinase A which MT1-MMP activates at sites of its expression is well correlated with the infiltration and metastasis of cancer.

Since MT5-MMP is expressed in undifferentiated-type glioma, glioma, pancreatic cancer and hepatoma cell lines, the possibility has been suggested that excessive expression of MT5-MMP is involved in the malignant nature of cancer cells in a specific types of cancers.

### EXAMPLE 17. Preparation of Polyclonal Antibodies that Recognize MT5-MMP

An MT5-MMP partial peptide expressed in *E*. *coli,* more specifically, 100 *µ* g of each of the MT5-MMP partial peptides represented by SEQ ID NOS: 23 to 27 synthesized in Example 19 below (Compounds 1 to 5) is administered to two rabbits (Japanese White Rabbits) together with complete Freund's adjuvant.

From two weeks after the initial administration, 100 *µ* g of the MT5-MMP partial peptide is administered once a week together with incomplete Freund's adjuvant 6 times in the total.

Blood samples are taken from the ear microvein. Then, serum antibody titers of the samples are examined by the enzyme immunoassay as described in (2) in Example 18 below. Then, serum is collected from rabbits that exhibited a sufficient antibody titer by exsanguination.

The resultant serum is purified to IgG fraction by the method as described in (5) in Example 18 below. This fraction is used as a polyclonal antibody.

### EXAMPLE 18. Preparation of Monoclonal Antibodies that Recognize MT5-MMP

### (1) Immunization of Animals and Preparation of Antibody-Producing Cells

An MT5-MMP partial peptide expressed in *E*. *coli*, more specifically, 50 *µ* g of each of the MT5-MMP partial peptides represented by SEQ ID NOS: 23 to 27 synthesized in Example 19 below (Compounds 1 to 5) is administered to 5-week old female SD rats together with 2 mg of aluminium hydroxide adjuvant and 1x10⁹ cells of pertussis vaccine (Serum Institute, Chiba Pref., Japan).

From two weeks after the initial administration, 50 *µ* g of the MT5-MMP partial peptide is administered once a week 4 times in the total.

Blood samples are taken from the venous plexus of the eyeground of the rats. Serum antibody titers of the samples are examined by the enzyme immunoassay as described below. Three days after the final immunization, the spleen is removed from rats that exhibited a sufficient antibody titer.

The spleen is cut into pieces in MEM medium (Nissui Pharmaceutical), loosened with tweezers and centrifuged (at 1,200 xg for 5 min).

To the resultant precipitate fraction, Tris-ammonium chloride buffer (pH 7.65) is added to treat it for 1 to 2 min. to thereby remove erythrocytes.

The resultant precipitate fraction (cell fraction) is washed with MEM medium 3 times and then used in cell fusion.

### (2) Enzyme Immunoassay

As antigens for this assay, the MT5-MMP partial peptides described above are used. As control antigens, human MT1-MMP hemopexin-like domain (hereinafter, abbreviated to "human MT1-MMP") is used.

Each of the antigens is dispensed at a concentration of 10 *µ* g/mL in a 50 *µ* L aliquot into all wells of 96-well ELISA plates, and left overnight at 4°C for adsorption.

After the plate is washed, 1% BSA-PBS is added thereto (100 *µ* L/well) and left at room temperature for one hour to thereby block the remaining active groups.

Subsequently, the 1 % BSA-PBS is discarded from the plate. An immunized rat antiserum, a culture supernatant of anti-MT5-MMP monoclonal antibody, or a purified monoclonal antibody is added to the plate (50 *µ* L/well) and left for two hours.

After the plate is washed with Tween-PBS, peroxidase-labeled rabbit anti-rat immunoglobulin (DAKO) is added thereto (50 *µ* L/well) and left at room temperature for one hour.

After the plate is washed with Tween-PBS, an ABTS substrate solution [ammonium 2,2-azino-bis(3-ethylbenzothiazol-6-sulfonate)] is added for color development. The absorbance at 415 nm (OD₄₁₅) is measured with a plate reader (Emax; Molecular Devices).

### (3) Preparation of Mouse Myeloma Cells

An 8-azaguanine resistant mouse myeloma cell line P3-U1 is cultured in a normal medium to secure 2x10⁷ cells or more at the time of cell fusion. This cell line is supplied to cell fusion as a parent cell line.

### (4) Preparation of Hybridomas

The rat spleen cells obtained in (1) above and the myeloma cells obtained in (3) above are mixed to give a ratio of 10:1 and centrifuged (at 1,200 rpm for 5 min).

Cell pellet in the resultant precipitate fraction is loosened thoroughly. Then, a liquid mixture composed of 2 g of polyethylene glycol-1000 (PEG-1000), 2 mL of MEM medium and 0.7 mL of dimethylsulfoxide is added to the cells in an amount of 0.2-1 mL per 108 rat spleen cells at 37°C while stirring. To the suspension, 1-2 mL of MEM medium is added every one to two minutes (several times in the total). Then, MEM medium was added thereto to make the total volume 50 mL.

This suspension is centrifuged (at 900 rpm for 5 min) to obtain a precipitate fraction. Cells in this fraction are loosened and then gently suspended in 100 mL of HAT medium by pipetting gently with a measuring pipette.

The resultant suspension is added to 96-well culture plates (100 *µ* L/well) and cultured in a 5% CO₂ incubator at 37°C for 10-14 days.

After the culturing, the culture supernatant is examined by the enzyme immunoassay described in (2) above to thereby select those wells that react with the MT5-MMP partial polypeptide but do not react with human MT1-MMP. Cells contained in the selected wells are subjected to cloning twice by limiting dilution technique to thereby establish anti-MT5-MMP monoclonal antibody-producing hybridomas.

### (5) Purification of Monoclonal Antibodies

Each of the hybridoma clones obtained in (4) above is administered to pristane-treated 8-week old nude female mice (Balb/c) by intraperitoneal injection (5-20 x 10⁶ cells/mouse).

Ten to 21 days after the administration, abdominal dropsy is collected (1-8 mL/mouse) from those mice in which abdominal dropsy has been accumulated as a result of the conversion of the hybridoma into abdominal dropsy cancer.

The abdominal dropsy is centrifuged (at 3,000 rpm for 5 min) to remove solid matter.

Purified IgM monoclonal antibodies are obtained by salting out the above abdominal dropsy with 50% ammonium sulfate, dialyzing against PBS to which 0.5 M sodium chloride has been added, and then applying to a column packed with Cellulofine GSL2000 (Seikagaku Corp.) (bed volume: 750 mL) at a flow rate of 15 mL/hr to thereby collect the IgM fraction.

Purified IgG monoclonal antibodies are obtained by purification using the caprylic acid precipitation technique [Antibodies - A Laboratory Manual, Cold Spring Harbor Laboratory, 1988].

The subclasses of the antibodies were determined by ELISA using a subclass typing kit.

### (6) Western Blotting

The reaction specificities of the anti-MT5-MMP monoclonal antibodies selected in (4) above are examined by Western blotting.

The MT5-MMP partial peptide or human MT1-MMP (1 *µ* g/lane) is fractionated by SDS-polyacrylamide electrophoresis [Antibodies - A Laboratory Manual, Cold Spring Harbor Laboratory, 1988] and blotted on a PVDF membrane.

After the membrane is blocked with BSA-PBS, a culture supernatant of the anti-MT5-MMP monoclonal antibody is added to the membrane and left at room temperature for 2 hr.

After the membrane is washed with Tween-PBS thoroughly, peroxidase-labeled anti-rat immunoglobulin antibody (DAKO) is added to the membrane as the secondary antibody and left at room temperature for one hour.

After the membrane is washed with Tween-PBS thoroughly, reaction is detected using Color Development Reagent (Bio-Rad) to thereby confirm that the anti-MT5-MMP monoclonal antibody reacts specifically with a band corresponding to the molecular weight of MT5-MMP polypeptide.

### EXAMPLE 19. Synthesis of Human MT5-MMP Partial Peptides

Human MT5-MMP partial peptides (Compounds 1 to 5) were synthesized based on the amino acid sequence as shown in SEQ ID NO: 6.

### Abbreviations:

The abbreviations for amino acids and protecting groups thereof used in the present invention are in accordance with the recommendations by the IUPAC-IUB Commission (IUPAC-IUB Joint Commission on Biochemical Nomenclature) [European Journal of Biochemistry, Vol. 138, p. 9, (1984)].

Unless otherwise indicated, the following abbreviations represent the amino acids indicated at the right, respectively.
- Ala:: L-Alanine
- Asn:: L-Asparagine
- Asp:: L-Aspartic acid
- Arg:: L-Arginine
- Cys:: L-Cysteine
- Gln:: L-Glutamine
- Glu:: L-Glutamic acid
- Gly:: L-Glycine
- His:: L-Histidine
- Ile:: L-Isoleucine
- Leu:: L-Leucine
- Lys:: L-Lysine
- Met:: L-Methionine
- Phe:: L-Phenylalanine
- Pro:: L-Proline
- Ser:: L-Serine
- Thr:: L-Threonine
- Trp:: L-Tryptophan
- Tyr:: L-Tyrosine
- Val:: L-Valine

The following abbreviations represent the amino acid protecting groups and side chain-protected amino acids indicated at the right, respectively.
- Fmoc:: 9-Fluorenylmethyloxycarbonyl
- Ac:: Acetyl
- tBu:: t-Butyl
- Boc:: t-Butyloxycarbonyl
- Trt:: Trityl
- Pmc:: 2,2,5,7,8-Pentamethylchroman-6-sulfonyl
- Fmoc-Thr(tBu)-OH:: N^{α}-9- Fluorenylmethyloxycarbonyl-O-t-butyl-L-threonine
- Fmoc-Ser(tBu)-OH:: N^{α}-9-Fluorenylmethyloxycarbonyl-O-t-butyl-L-serine
- Fmoc-Tyr(tBu)-OH:: N^{α}-9-Fluorenylmethyloxycarbonyl-O-t-butyl-L-tyrosine
- Fmoc-Lys(Boc)-OH:: N^{α}-9-Fluorenylmethyloxycarbonyl-N^{ε}-t-butyloxycarbonyl-L-lysine
- Fmoc-Glu(OtBu)-OH:: N^{α}-9-Fluorenylmethyloxycarbonyl-L-glutamic acid- γ -t-butyl ester
- Fmoc-Arg(Pmc)-OH:: N^{α}-9-Fluorenylmethyloxycarbonyl-N^{g}-2,2,5,7,8-pentamethylchroman-6-sulfonyl-L-arginine
- Fmoc-His(Trt)-OH:: N^{α}-9-Fluorenylmethyloxycarbonyl-N^{im}-trityl-L-histidine
- Fmoc-Trp(Boc)-OH:: N^{α}-9-Fluorenylmethyloxycarbonyl-N^{ind}-t-butyloxycarbonyl-L-tryptophan
- Fmoc-Asn(Trt) -OH:: N^{α}-9-Fluorenylmethyloxycarbonyl-N^{β}-trityl-L-asparagine
- Fmoc-Asp(OtBu)-OH:: N^{α}-9-Fluorenylmethyloxycarbonyl-L-aspartic acid- β -t-butyl ester
- Fmoc-Gln(Trt)-OH:: N^{α}-9-Fluorenylmethyloxycarbonyl-N⁷ -trityl-L-glutamine
- Fmoc-Cys(Trt)-OH:: N^{α}-9-Fluorenylmethyloxycarbonyl-S-trityl-L-cysteine

The following abbreviations represent the reaction solvents or reaction reagents indicated at the right, respectively.
- HBTU:: 2-(1H-Benzotriazole-1-yl)-1,1,3,3-tetramethyluronium hexafluorophosphate
- HOBt:: N-Hydroxybenzotriazole
- DMF:: N,N-Dimethylformamide
- TFA:: Trifluoroacetic acid
- DIEA:: Diisopropylethylamine

### (1) Synthesis of Compound 1 (SEQ ID NO: 23) : Ac-Pro-Val-Thr-Gly-Val-Leu-Asp-Gln-Thr-Thr-Ile-Glu-Trp-Met-Lys-Lys-Cys-OH

Thirty milligrams of a support resin to which 16.8 *µ mol* of H-Cys(Trt) had been linked (Cl-Trt resin; manufactured by Ana Spec) was put in a reactor of an automated synthesizer (Shimadzu Corp). One milliliter of DMF was added thereto and agitated for 10 min, and then the DMF solution was discharged. Subsequently, the following operations were performed according to the synthesis program of Shimadzu Corp.
(a) Fmoc-Lys(Boc)-OH (200 *µ* mol), HBTU (200 *µ* mol), HOBt monohydrate (200 *µ* mol) and DIEA (400 *µ* mol) were agitated in 800 *µ* L of DMF for 3 min. The resultant solution was added to the support resin. The mixture was agitated for 30 min, and then the solution was discharged.
(b) The support resin was washed in 900 *µ* L of DMF for 1 min. This washing was repeated 5 times to thereby link Fmoc-Lys(Boc)-Cys(Trt) to the support resin.
   Subsequently, the Fmoc group deprotection step described below was carried out.
(c) To the resultant support resin, 900 *µ* L of 30% piperidine-DMF solution was added and agitated for 4 min, and then the solution was discharged. This operation was repeated again.
(d) The support resin was washed in 600 *µ* L of DMF for 1 min, and then the DMF solution was discharged. This operation was repeated 5 times.

By these procedures, a support resin to which Fmoc group-removed H-Lys(Boc)-Cys(Trt) had been linked was obtained. Subsequently, condensation reaction was carried out in step (a) using Fmoc-Lys(Boc)-OH; washing was carried out in step (b); and deprotection was carried out in steps (c) and (d). Through these processes, H-Lys(Boc)-Lys(Boc)-Cys(Trt) was synthesized on the support resin. Subsequently, using Fmoc-Met-OH, Fmoc-Trp(Boc)-OH, Fmoc-Glu(OtBu)-OH, Fmoc-Ile-OH, Fmoc-Thr(tBu)-OH, Fmoc-Thr(tBu)-OH, Fmoc-Gln(Trt)-OH, Fmoc-Asp(OtBu)-OH, Fmoc-Leu-OH, Fmoc-Val-OH, Fmoc-Gly-OH, Fmoc-Thr(tBu)-OH, Fmoc-Val-OH and Fmoc-Pro-OH in step (a) in order, steps (a) to (d) were repeated.

Then, an acetyl group was introduced to the N-terminus of the resultant sequence using 32 *µ* L of acetic anhydride and 900 *µ* L of DMF. Subsequently, the support resin was washed with DMF, methanol and butyl ether in order and dried under reduced pressure to yield a support resin to which a side chain-protected peptide had been linked. To this support resin, 1 mL of a mixed solution composed of TFA (90%), thioanisole (5%) and 1,2-ethanedithiol (5%) was added and left at room temperature for 2 hr to thereby remove the side-chain protecting group and to cut out the peptide from the resin. After removal of the resin by filtration, approximately 10 mL of ether was added to the filtrate. The resultant precipitate was recovered by centrifugation and decantation and then dried under reduced pressure to obtain 42.1 mg of crude peptide. This crude product was diluted with 5 mL of 90% acetic acid and purified by HPLC using a reversed-phase column (Shiseido; CAPCELL PAK C18, 30 mm I.D. x 25 mm). Then, the adsorbate was eluted by the linear concentration gradient method in which 0.1% TFA-containing aqueous solution of 90% acetonitrile was gradually added to aqueous solution of 0.1% TFA. As a result of detection at 220 nm, a fraction containing Compound 1 was obtained. This fraction was freeze-dried to yield 3.2 mg of Compound 1.
Mass spectrometric analysis [FABMS]: m/z =1991.7 (M+H⁺)
Amino acid analysis: Asx 1.1 (1), Glx2.1(2), Glyl.1(1), Thr 2.9(3), Pro 1.0 (1), Val 1.8(2), Met 1.1(1), Lys2.0(2), Ile1.0(1), Leu1.0(1), Cys1.1(1)

### (2) Synthesis of Compound 2 (SEQ ID NO: 24): Ac-His-Glu-Ile-Lys-Ser-Asp-Arg-Lys-Glu-Ala-Asp-Ile-Met-Ile-Phe-Phe-Ala-Ser-Cys-OH

Using 30 mg of a support resin to which 16.8 *µ* mol of H-Cys(Trt) had been linked (Cl-Trt resin; manufactured by Ana Spec) as a starting material, Fmoc-Ser(tBu)-OH, Fmoc-Ala-OH, Fmoc-Phe-OH, Fmoc-Phe-OH, Fmoc-Ile-OH, Fmoc-Met-OH, Fmoc-Ile-OH, Fmoc-Asp(OtBu)-OH, Fmoc-Ala-OH, Fmoc-Glu(OtBu)-OH, Fmoc-Lys(Boc)-OH, Fmoc-Arg(Pmc)-OH, Fmoc-Asp(OtBu)-OH, Fmoc-Ser(tBu)-OH, Fmoc-Lys(Boc)-OH, Fmoc-Ile-OH, Fmoc-Glu(OtBu)-OH, and Fmoc-His(Trt)-OH were condensed in order in the same manner as described in (1) above. Then, an acetyl group was introduced to the N-terminus of the resultant sequence using acetic anhydride. Through washing and drying, a support resin to which a side chain-protected peptide had been linked was obtained. To this support resin, 1 mL of a mixed solution composed of TFA (82.5%), thioanisole (5%), water (5%) ethyl methyl sulfide (3%), 1,2-ethanedithiol (2.5%) and thiophenol (2%) was added and left at room temperature for 8 hr to thereby remove the side-chain protecting group and to cut out the peptide from the resin. Subsequently, 38 mg of a crude peptide was obtained and purified by HPLC using a reversed-phase column in the same manner as described in (1) above. As a result, 3.1 mg of Compound 2 was obtained.
Mass spectrometric analysis [FABMS]: m/z = 2283.3 (M+H⁺)
Amino acid analysis: Asx 2.1 (2), Glx 2.2 (2), Ser 1.7(2), His 0.9(1), Arg 1.0(1), Ala 2.2 (2), Met 1.0(1), Lys2.0(2), Ile 2.9(3), Phe 2.1(2), Cys 1.2(1)

### (3) Synthesis of Compound 3 (SEQ ID NO: 25) : Ac-Leu-Pro-Val-Arg-Arg-Ile-His-Ser Pro-Ser-Glu-Arg-Lys-His-Glu-Arg-Gln-Cys-OH

Using 30 mg of a support resin to which 16.8 *µ* mol of H-Cys(Trt) had been linked (Cl-Trt resin; manufactured by Ana Spec) as a starting material, Fmoc-Gln(Trt)-OH, Fmoc-Arg(Pmc)-OH, Fmoc-Glu(OtBu)-OH, Fmoc-His(Trt)-OH, Fmoc-Lys(Boc)-OH, Fmoc-Arg(Pmc)-OH, Fmoc-Glu(OtBu)-OH, Fmoc-Ser(tBu)-OH, Fmoc-Pro-OH, Fmoc-Ser(tBu)-OH, Fmoc-His(Trt)-OH, Fmoc-Ile-OH, Fmoc-Arg(Pmc)-OH, Fmoc-Arg(Pmc)-OH, Fmoc-Val-OH, Fmoc-Pro-OH, and Fmoc-Leu-OH were condensed in order in the same manner as described in (1) above. Then, an acetyl group was introduced to the N-terminus of the resultant sequence using acetic anhydride. Through washing and drying, a support resin to which a side chain-protected peptide had been linked was obtained. To this support resin, 1 mL of a mixed solution composed of TFA (82.5%), thioanisole (5%), water (5%) ethyl methyl sulfide (3%), 1,2-ethanedithiol (2.5%) and thiophenol (2%) was added and left at room temperature for 8 hr to thereby remove the side-chain protecting group and to cut out the peptide from the resin. Subsequently, 49.2 mg of a crude peptide was obtained and purified by HPLC using a reversed-phase column in the same manner as described in (1) above. As a result, 20.4 mg of Compound 3 was obtained.
Mass spectrometric analysis [FABMS] : m/z = 2271.4 (M+H⁺)
Amino acid analysis: Glx 2.9 (3), Ser 2.0(2), His 1.9(2), Arg 4.0(4), Pro 2.2 (2), Val 1.0(1), Lys 1.1 (1), Ile 0.8(1), Leu 1.1(1), Cys 1.2(1)

### (4) Synthesis of Compound 4 (SEQ ID NO: 26) : H-Cys-Asn-Gln-Lys-Glu-Val-Glu-Arg-Arg-Lys-Glu-Arg-Arg-Leu-Pro-Gln-Asp-NH₂

Using 30 mg of a support resin to which 16.5 *µ* mol of Fmoc-NH had been linked (RINK Amide MBHA resin; manufactured by Nova Biochem) as a starting material, Fmoc-Asp(OtBu)-OH, Fmoc-Gln(Trt)-OH, Fmoc-Pro-OH, Fmoc-Leu-OH, Fmoc-Arg(Pmc)-OH, Fmoc-Arg(Pmc)-OH, Fmoc-Glu(OtBu)-OH, Fmoc-Lys(Boc)-OH, Fmoc-Arg(Pmc)-OH, Fmoc-Arg(Pmc)-OH, Fmoc-Glu(OtBu)-OH, Fmoc-Val-OH, Fmoc-Glu(OtBu)-OH, Fmoc-Lys(Boc)-OH, Fmoc-Gln(Trt)-OH, Fmoc-Asn(Trt)-OH, and Fmoc-Cys(Trt)-OH were condensed in order in the same manner as described in (1) above. Through washing and drying, a support resin to which a side chain-protected peptide had been linked was obtained. To this support resin, I mL of a mixed solution composed of TFA (82.5%), thioanisole (5%), water (5%) ethyl methyl sulfide (3%), 1,2-ethanedithiol (2.5%) and thiophenol (2%) was added and left at room temperature for 8 hr to thereby remove the side-chain protecting group and to cut out the peptide from the resin. Subsequently, 52.8 mg of a crude peptide was obtained and purified by HPLC using a reversed-phase column in the same manner as described in (1) above. As a result, 21.3 mg of Compound 4 was obtained.
Mass spectrometric analysis [FABMS] : m/z = 2183.6 (M+H⁺)
Amino acid analysis: Asx 1.9 (2), Glx 5.1(5), Arg 3.9(4), Pro 1.1(1), Val 1.0(1), Lys 2.0 (2), Leu 1.0 (1), Cys 0.8 (1)

### (5) Synthesis of Compound 5 (SEQ ID NO: 27): H-Cys-Asn-Lys-Thr-Gly-Pro-Gln-Pro-Val-Thr-Tyr-Tyr-Lys-Arg-Pro-Val-Gln-Glu-Trp-Val-OH

Using 30 mg of a support resin to which 15.6 *µ* mol of Fmoc-Val had been linked (Wang resin; manufactured by Shimadzu Corp.) as a starting material, Fmoc-Trp(Boc)-OH, Fmoc-Glu(OtBu)-OH, Fmoc-Gln(Trt)-OH, Fmoc-Val-OH, Fmoc-Pro-OH, Fmoc-Arg(Pmc)-OH, Fmoc-Lys(Boc)-OH, Fmoc-Tyr(tBu)-OH, Fmoc-Tyr(tBu)-OH, Fmoc-Thr(tBu)-OH, Fmoc-Val-OH, Fmoc-Pro-OH, Fmoc-Gln(Trt)-OH, Fmoc-Pro-OH, Fmoc-Gly-OH, Fmoc-Thr(tBu)-OH, Fmoc-Lys(Boc)-OH, Fmoc-Asn(Trt)-OH, and Fmoc-Cys(Trt)-OH were condensed in order in the same manner as described in (1) above. Through washing and drying, a support resin to which a side chain-protected peptide had been linked was obtained. To this support resin, 1 mL of a mixed solution composed of TFA (82.5%), thioanisole (5%), water (5%) ethyl methyl sulfide (3%), 1,2-ethanedithiol (2.5%), thiophenol (2%) and 2-methylindole (5 mg/mL) was added and left at room temperature for 6 hr to thereby remove the side-chain protecting group and to cut out the peptide from the resin. Subsequently, 52.8 mg of a crude peptide was obtained and purified by HPLC using a reversed-phase column in the same manner as described in (1) above. As a result, 16.2 mg of Compound 5 was obtained.
Mass spectrometric analysis [FABMS] : m/z = 2394.3 (M+H⁺)
Amino acid analysis: Asx 1.0 (1), Glx 3.1 (3), Gly 1.0(1), Arg 1.0(1), Thr 1.9(2), Pro 3.1 (3), Tyr2.0(2), Val 3.0(3), Lys 1.9 (2), Cys 1.1(1)

### EXAMPLE 20. Preparation of Polyclonal Antibodies that Recognize Human MT5-MMP

### (1) Preparation of Immunogen

Compounds 1 to 5 that are partial peptides of the human MT5-MMP polypeptide obtained in Example 19 were conjugated with KLH (Calbiochem) by the following method for the purpose of enhancing their immunogenicity. The resultant conjugates were used as immunogens. Briefly, KLH was dissolved in PBS to give a concentration of 10 mg/mL. To this solution, 1/10 volume of 25 mg/mL MBS [N-(m-maleimidobenzoyloxy) succinimide; Nacalai Tesque] was added in drops and reacted for 30 min under agitation. Two point five milligrams of KLH-MB that had been obtained by removing free MBS through a gel filtration column such as SephadexG-25 column pre-equilibrated with PBS was mixed with 1 mg of the above peptide dissolved in 0.1 M sodium phosphate buffer (pH 7.0) and reacted at room temperature for 3 hr under agitation. After the reaction, the reaction product was dialyzed against PBS and used as an immunogen.

### (2) Immunization of Animals and Preparation of Polyclonal Antibodies

KLH-conjugated Compounds 1, 2 and 4 prepared in (1) above were mixed in equal amounts, and 200 *µ* g of this mixture was administered to two rabbits (Japan White Rabbits) together with complete Freund's adjuvant. From two weeks after the initial administration, 200 *µ* g of the mixed KLH conjugate (with Compounds 1, 2 and 4) was administered once a week together with incomplete Freund's adjuvant 7 times in the total.

Blood samples were taken from the ear microvein. Then, serum antibody titers of the samples were examined by the enzyme immunoassay as described in (2) in Example 21 below. Then, serum was collected from rabbits that exhibited a sufficient antibody titer by exsanguination. The resultant serum was purified to IgG fraction by the method as described in (5) in Example 21 below. This fraction was used as a polyclonal antibody.

The reactivity of the thus obtained polyclonal antibody with Compounds 1, 2 and 4 was examined by the enzyme immunoassay described in (2) in Example 21 below. As a result, as shown in Fig. 7, specific increase in antibody titer was observed against Compounds 1 and 2 in both lot 1 and lot 2. No increase in antibody titer was observed against Compound 4.

### EXAMPLE 21. Preparation of Monoclonal Antibodies that Recognize Human MT5-MMP

### (1) Immunization of Animals and Preparation of Antibody-Producing Cells

One hundred micrograms of each of the KLH-conjugated Compounds 1 to 5 prepared in Example 19 was administered to three 5-week old female BALB/c mice together with 2 mg of aluminium hydroxide adjuvant (Antibodies - A Laboratory Manual, Cold Spring Harbor Laboratory, p. 99, 1988) and 1x10⁹ cells of pertussis vaccine (Serum Institute, Chiba Pref., Japan). From two weeks after the initial administration, 100 *µ* g of each KLH-conjugated compound was administered once a week 4 times in the total. Blood samples were taken from the venous plexus of the eyeground of the mice. Then, serum antibody titers of the samples were examined by the enzyme immunoassay as described below. Three days after the final immunization, the spleen was removed from those mice that exhibited a sufficient antibody titer.

The spleen was cut into pieces in MEM medium (Nissui Pharmaceutical), loosened with tweezers and centrifuged (at 250 xg for 5 min). To the resultant precipitate fraction, Tris-ammonium chloride buffer (pH 7.6) was added to treat it for 1 to 2 min to thereby remove erythrocytes. The resultant precipitate fraction (cell fraction) was washed with MEM medium 3 times and then used in cell fusion.

### (2) Enzyme Immunoassay (Binding ELISA)

As antigens for this assay, the Compounds obtained in Example 19 conjugated with thyroglobulin (hereinafter, abbreviated to THY) were used. The method of preparation of these conjugates was as described in (1) in Example 20 except that SMCC [4-(N-maleimidomethyl)-cyclohexane-1-carboxylic acid N-hydroxysuccinimido ester; Sigma] was used as a cross-linking agent instead of MBS. Each of the conjugates was dispensed at a concentration of 10 *µ* g/mL and added to all wells of 96-well ELISA plates (50 *µ* L/well), and left overnight at 4°C for adsorption. After the plate was washed, 1 % bovine serum albumin (BSA)/Dulbecco's phosphate buffer (phosphate buffered saline: PBS) was added thereto (100 *µ* L/well) and left at room temperature for one hour to thereby block the remaining active groups.

Subsequently, the 1% BSA/PBS was discarded from the plate. An immunized mouse anti-serum, a culture supernatant of anti-human MT5-MMP monoclonal antibody, or a purified monoclonal antibody was added to the plate (50 *µ* L/well) and left for two hours. After the plate was washed with 0.05% polyoxyethylene (20) sorbitan monolaurate [equivalent to Tween™ 20 of ICI; manufactured by Wako Purechemical Industries]/PBS (hereinafter, referred to as Tween-PBS), peroxidase-labeled rabbit anti-mouse immunoglobulin was added (50 *µ* L/well) and then left at room temperature for one hour. When an immunized_rabbit antiserum or an IgG fraction was used, they were reacted in the same manner and then a 200-fold dilution of peroxidase-labeled pig anti-rabbit immunoglobulin (DAKO) was added as the secondary antibody. After the plate was washed with Tween-PBS, an ABTS [ammonium 2,2-azino-bis(3-ethylbenzothiazol-6-sulfonate)] substrate solution [I mmol/L ABTS/0.1 mol/L citrate buffer (pH 4.2)] was added for color development. The absorbance at 415 nm (OD₄₁₅) was measured with a plate reader (Emax; Molecular Devices).

### (3) Preparation of Mouse Myeloma Cells

An 8-azaguanine resistant mouse myeloma cell line P3X63Ag8U.1 (P3-U1; purchased from ATCC) was cultured in a normal medium (10% fetal bovine serum-added RPMI medium) to secure 2x10⁷ cells or more at the time of cell fusion. This cell line was supplied to cell fusion as a parent cell line.

### (4) Preparation of Hybridomas

The mouse spleen cells obtained in (1) above and the myeloma cells obtained in (3) above were mixed to give a ratio of 10:1 and centrifuged (at 250 xg for 5 min). Cell pellet in the resultant precipitate fraction was loosened thoroughly. Then, a liquid mixture composed of 2 g of polyethylene glycol-1000 (PEG-1000), 2 mL of MEM medium and 0.7 mL of dimethylsulfoxide was added to the cells in an amount of 0.5 mL per 10⁸ mouse spleen cells at 37°C while stirring. To the suspension, 1 mL of MEM medium was added every one to two minutes (several times in the total). Then, MEM medium was added thereto to make the total volume 50 mL.

This suspension was centrifuged (at 900 rpm for 5 min) to obtain a precipitate fraction. Cells in this fraction were loosened and then gently suspended in 100 mL of HAT medium [10% fetal bovine serum-added RPMI medium to which HAT Media Supplement (Boehringer Mannheim) was added] by pipetting gently with a measuring pipette. The resultant suspension was added to 96-well culture plates (200 *µ* L/well) and cultured in a 5% CO₂ incubator at 37°C for 10-14 days.

After the culturing, the culture supernatant was examined by the enzyme immunoassay described in (2) above to thereby select those wells that reacted with the antigen peptide but did not react with the control peptide. Cells contained in the selected wells were subjected to cloning twice by limiting dilution technique. As a result, anti-human MT5-MMP monoclonal antibody-producing hybridomas were established. Hybridomas KM2645-2655 were obtained using Compound 3 as the antigen, and hybridomas KM2656-2661 were obtained using Compound 5 as the antigen.

Hybridomas KM2655 and KM2658 were deposited as FERM BP-6883 and FERM BP-6884, respectively, at the National Institute of Bioscience and Human-Technology, Agency of Industrial Science and Technology (1-3, Higashi 1-Chome, Tsukuba City, Ibaraki Pref., Japan) on September 21, 1999.

As shown in Fig. 8, the monoclonal antibody produced by any of the hybridomas indicated specific reactivity with the compound used as the antigen.

### (5) Purification of Monoclonal Antibodies

Each of the hybridoma clones obtained in (4) above was administered to pristane-treated 8-week old nude female mice (BALB/c) by intraperitoneal injection (5-20 x 10⁶ cells/mouse). Ten to 21 days after the administration, abdominal dropsy was collected (1-8 mL/mouse) from those mice in which abdominal dropsy had been accumulated as a result of the conversion of the hybridoma into abdominal dropsy cancer.

The abdominal dropsy was centrifuged (at 1,200 xg for 5 min) to remove solid matter.

Purified IgG monoclonal antibodies were obtained by purification using the caprylic acid precipitation technique [Antibodies - A Laboratory Manual, Cold Spring Harbor Laboratory, 1988]. The subclasses of the antibodies were determined by ELISA using a subclass typing kit. The results are shown in Table 4.

**Table 4.**

| Antibody Classes of Anti-Human MT5-MMP Monoclonal Antibody-Producing Hybridomas KM2645-2661 | |
|---|---|
| KM No. | Subclass |
| KM2645 | G1 |
| KM2646 | G1 |
| KM2647 | G1 |
| KM2648 | G2b |
| KM2649 | G2b |
| KM2650 | G2b |
| KM2651 | G1 |
| KM2652 | G2b |
| KM2653 | G2b |
| KM2654 | G2a |
| KM2655 | G1 |
| KM2656 | G1 |
| KM2657 | G1 |
| KM2658 | G1 |
| KM2659 | G1 |
| KM2660 | G1 |
| KM2661 | G1 |

### EXAMPLE 22. Detection of Human MT5-MMP Protein using Anti-Human MT5-MMP Antibody

### (1) Transfer of Human MT5-MMP Gene into COS-1 Cell Line

First, a FLAG epitope (DYKDDDDK; SEQ ID NO: 28) was inserted at the N-terminus of the catalytic domain of human MT5-MMP gene by PCR extension technique. The resultant human MT5-MMP-FLAG gene was subcloned into pTLl Vector (an expression vector) [Stratagene; pSG5 Vector into which SacI, KphI and SmaI restriction sites are introduced] and then transferred into *E. coli* XL-1 Blue MRF'. This *E. coli* was cultured in 150 mL of LB (Luria-Bertani) medium in the presence of 100 *µ* g/mL ampicillin followed by purification of the plasmid DNA using the AX500 cartridge of NucleoBond Plasmid Kit (Clontech).

As a host cell for gene transfer, simian kidney-derived COS-1 cells (ATCC CRL-1650) purchased from ATCC were used. Five milliliters of cultured COS-1 cells adjusted to give a concentration of 2x10⁵ cells/mL were added to a 5 cm culture dish and cultured overnight. Then, using FuGENE™6 transfection reagent (Boehringer Mannheim), gene transfer was carried out as described below.

Briefly, a serum-free medium OPTI-MEM^{R}I (Lifetech Oriental) (408 *µ* L) was placed in a plastic tube, to which 12 *µ* L of FuGENE was added and left stationary at room temperature for 5 min. In another plastic tube, 6 *µ* L of a solution of the above-described plasmid DNA from human MT5-MMP diluted to 1 *µ* g/*µ* L with Tris-EDTA (pH 8.0) was added. Then, the FuGENE solution prepared above was added thereto, mixed gently and left stationary for 15 min. This plasmid solution (426 *µ* L) was added to the culture of COS-1 cell line that had been cultured since the previous day. After the culture liquid was made homogeneous, the cells were cultured for 3 days. Human MT4-MMP(2) gene was also transferred in a similar manner.

### (2) Detection of Human MT5-MMP Protein by Western Blotting

MT5-MMP gene-transferred COS-1 cells prepared as described in (1) above and untreated COS-1 cells were collected by pipetting. After the cells were washed with PBS once, an SDS-polyacrylamide electrophoresis (SDS-PAGE) sample buffer [0.06 mol/L Tris-HCl (pH 6.8), 2% SDS, 10% glycerol, 5% 2-mercaptoethanol] was added to the cells to give a concentration of 1x10⁷ cells/mL, heated at 100°C for 5 min and then sonicated to thereby solubilize the cells completely.

The thus prepared solution of solubilized cells [10 *µ* L (1x10⁵ cells)/lane] was separated by SDS-polyacrylamide electrophoresis (SDS-PAGE: 5-20% gradient gel; Atto) [Antibodies - A Laboratory Manual, Cold Spring Harbor Laboratory, 1988] and blotted on a PVDF membrane (Millipore).

After the membrane was blocked with 1% BSA-PBS, undiluted culture supernatant of the anti-human MT5-MMP monoclonal antibody was added to the membrane and left at room temperature for 2 hr. After the membrane was washed with Tween-PBS thoroughly, a 2000-fold dilution of peroxidase-labeled rabbit anti-mouse immunoglobulin antibody (DAKO) was added as the secondary antibody and left at room temperature for 1 hr.

After the membrane was washed with Tween-PBS thoroughly, reaction was detected using ECL kit (Amersham Pharmacia Biotech). As control antibodies, anti-mouse MT4-MMP monoclonal antibody KM2561 and anti-FLAG monoclonal antibody (M2; Eastman Kodak Company) were reacted at a concentration of 10 *µ* g/mL and detected in the same manner.

As shown in Fig. 9, anti-human MT5-MMP monoclonal antibodies (KM2655 and KM2658) specifically reacted with a band around 66 kDa that corresponds to the molecular weight of human MT5-MMP protein.

### (3) Detection of Human MT5-MMP Protein by Fluorescein Antibody Technique (Flow Cytometry)

MT5-MMP gene-transferred COS-1 cells prepared as described in (1) above and untreated COS-1 cells were collected by pipetting. After washed with PBS, the cells were treated with ice-cooled 100% methanol at 4°C for 10 min to increase the antibody permeability of cell membranes. After washed with PBS, the cells were blocked with 10% normal rabbit serum at 4°C for 30 min. Then, the cells were dispensed into tubes (1x10⁵ cells/tube) and centrifuged to remove the supernatant. To the precipitate, a culture supernatant of anti-human MT5-MMP monoclonal antibody was added and reacted at 4°C for 30 min. After the cells were washed with PBS, a 30-fold dilution of FITC-labeled anti-mouse immunoglobulin antibody (specific to mouse immunoglobulin; Wako Purechemical Industries) were dispensed into tubes (100 *µ* L/tube) and reacted at 4°C for 30 min while shielding from light. After washed PBS thoroughly, the cells were analyzed with a cell analyzer (Coulter; EPICS XL system II). As a control antibody, anti-FLAG monoclonal antibody or anti-G-CSF derivative monoclonal antibody KM511 was reacted at a concentration of 10 *µ* g/mL and detected in the same manner.

As shown in Figs. 10 through 12, KM2648 and KM2652-2655 obtained from Compound 3 and KM2658 obtained from Compound 5 specifically detected human MT5-MMP protein expressed in COS-1 cells. The axis of ordinates represents cell count, and the axis of abscissas represents fluorescence intensity.

### SEQUENCE LISTING FREE TEXT

- SEQ ID NO: 23:: a partial amino acid sequence of MT5-MMP; Xaa = N^{α}-acetylproline
- SEQ ID NO: 24:: a partial amino acid sequence of MT5-MMP; Xaa = N^{α}-acetylhistidine
- SEQ ID NO: 25:: a partial amino acid sequence of MT5-MMP; Xaa = N^{α}-acetylleucine
- SEQ ID NO: 26:: a partial amino acid sequence of MT5-MMP
- SEQ ID NO: 27:: a partial amino acid sequence of MT5-MMP
- SEQ ID NO: 28:: FLAG epitope

### INDUSTRIAL APPLICABILITY

By using the antibodies to the novel MT4-MMP(2) polypeptide obtained by the present invention, it becomes possible to diagnose, prevent or treat diseases such as arthrosis deformans, rheumatoid arthritis, asthma, autoimmune diseases, atopic dermatitis, psoriasis, contact dermatitis, alopecia, ischemic heart diseases, immune response associated with organ transplantation, hepatitis, nephritis, pancreatitis, arteriosclerosis, leukemia, malignant tumor, wounds, corneal ulcer, tissue injury or inflammations associated with infiltration of leukocytes.

Further, by using the antibodies to the novel MT5-MMP polypeptide obtained by the present invention, it becomes possible to diagnose, prevent or treat diseases such as arthrosis deformans, rheumatoid arthritis, asthma, autoimmune diseases, atopic dermatitis, psoriasis, contact dermatitis, alopecia, ischemic heart diseases, immune response associated with organ transplantation, hepatitis, nephritis, pancreatitis, arteriosclerosis, leukemia, malignant tumor, wounds, corneal ulcer, tissue injury, inflammations associated with infiltration of leukocytes, brain disorders at the time of cerebral apoplexy, Alzheimer's disease, dementia, multiple sclerosis, Parkinson's disease or brain tumor.

## Claims

1. An antibody which recognizes a polypeptide consisting of the amino acid sequence as shown in SEQ ID NO: 1.

2. An antibody which recognizes a polypeptide consisting of an amino acid sequence wherein one or several amino acids are deleted, substituted or added in the amino acid sequence of the polypeptide according to claim 1 and having metalloproteinase activity.

3. An antibody which recognizes a polypeptide consisting of the amino acid sequence as shown in SEQ ID NO: 2.

4. An antibody which recognizes a polypeptide consisting of an amino acid sequence wherein one or several amino acids are deleted, substituted or added in the amino acid sequence of the polypeptide according to claim 3 and having metalloproteinase activity

5. An antibody which recognizes a polypeptide consisting of the amino acid sequence as shown in SEQ ID NO: 5.

6. An antibody which recognizes a polypeptide consisting of an amino acid sequence wherein one or several amino acids are deleted, substituted or added in the amino acid sequence of the polypeptide according to claim 5 and having metalloproteinase activity

7. An antibody which recognizes a polypeptide consisting of the amino acid sequence as shown in SEQ ID NO: 6.

8. An antibody which recognizes a polypeptide consisting of an amino acid sequence wherein one or several amino acids are deleted, substituted or added in the amino acid sequence of the polypeptide according to claim 7 and having metalloproteinase activity

9. A method of immunological detection of the polypeptide according to any one of claims 1 to 8, which comprises using the antibody according to any one of claims I to 8.

10. The method of immunological detection according to claim 9, wherein the method is selected from the group consisting of fluorescent antibody technique, enzyme-linked immunosorbent assay, radioimmunoassay, immunohistochemical staining, Western blotting, immunoprecipitation, enzyme immunoassay and sandwich ELISA.

11. A method of immunological quantitative determination of the polypeptide according to any one of claims 1 to 8, which comprises using the antibody according to any one of claims 1 to 8.

12. The method of immunological quantitative determination according to claim 11, wherein said method is an immunological detection method selected from the group consisting of fluorescent antibody technique, enzyme-linked immunosorbent assay, radioimmunoassay, immunohistochemical staining, Western blotting, immunoprecipitation, enzyme immunoassay and sandwich ELISA.

13. A diagnostic agent, therapeutic agent or prophylactic agent for arthrosis deformans, rheumatoid arthritis, asthma, autoimmune diseases, atopic dermatitis, psoriasis, contact dermatitis, alopecia, ischemic heart diseases, immune response associated with organ transplantation, hepatitis, nephritis, pancreatitis, arteriosclerosis, leukemia, malignant tumor, wounds, corneal ulcer, tissue injury or inflammations associated with infiltration of leukocytes, wherein said agent comprises the antibody according to any one of the claims 1 to 4.

14. A method of screening for a compound that modulates the expression of the polypeptide according to any one of claims 1 to 4, which comprises contacting a cell expressing the polypeptide with a test sample and determining the amount of the polypeptide using the antibody according to any one of claims 1 to 4.

15. A method of screening for a compound that binds to the polypeptide according to any one of claims 1 to 4, which comprises contacting the polypeptide with a test sample and then determining the amount of the antibody according to any one of claims 1 to 4 which can bind to the polypeptide.

16. A diagnostic agent, therapeutic agent or prophylactic agent for arthrosis deformans, rheumatoid arthritis, asthma, autoimmune diseases, atopic dermatitis, psoriasis, contact dermatitis, alopecia, ischemic heart diseases, immune response associated with organ transplantation, hepatitis, nephritis, pancreatitis, arteriosclerosis, leukemia, malignant tumor, wounds, corneal ulcer, tissue injury or inflammations associated with infiltration of leukocytes, wherein said agent comprises the compound obtained by the method according to claim 14 or 15.

17. A diagnostic agent, therapeutic agent or prophylactic agent for arthrosis deformans, rheumatoid arthritis, asthma, autoimmune diseases, atopic dermatitis, psoriasis, contact dermatitis, alopecia, ischemic heart diseases, immune response associated with organ transplantation, hepatitis, nephritis, pancreatitis, arteriosclerosis, leukemia, malignant tumor, wounds, corneal ulcer, tissue injury, inflammations associated with infiltration of leukocytes, brain disorders at the time of cerebral apoplexy, Alzheimer's disease, dementia, multiple sclerosis, Parkinson's disease or brain tumor, wherein said agent comprises the antibody of any one of claims 5 to 8.

18. A method of screening for a compound that modulates the expression of the polypeptide according to any one of claims 5 to 8, which comprises contacting a cell expressing the polypeptide with a test sample and determining the amount of the polypeptide using the antibody according to any one of claims 5 to 8.

19. A method of screening for a compound that binds to the polypeptide according to any one of claims 5 to 8, which comprises contacting the polypeptide with a test sample and then determining the amount of the antibody according to any one of claims 5 to 8 which binds to the polypeptide.

20. A diagnostic agent, therapeutic agent or prophylactic agent for arthrosis deformans, rheumatoid arthritis, asthma, autoimmune diseases, atopic dermatitis, psoriasis, contact dermatitis, alopecia, ischemic heart diseases, immune response associated with organ transplantation, hepatitis, nephritis, pancreatitis, arteriosclerosis, leukemia, malignant tumor, wounds, corneal ulcer, tissue injury, inflammations associated with infiltration of leukocytes, brain disorders at the time of cerebral apoplexy, Alzheimer's disease, dementia, multiple sclerosis, Parkinson's disease or brain tumor, wherein said agent comprises the compound obtained by the method according to claim 18 or 19.
